# EUROPEAN PATENT SPECIFICATION

(11) **EP 3 016 671 B1**
(45) Date of publication and mention of the grant of the patent: **08.09.2021**
(21) Application number: 14738785.6
(22) Date of filing: 03.07.2014
(51) Int. Cl.: A61K 38/12, C07K 16/28, G01N 33/68

(54) **ACTIVELY TRANSPORTED AND PROTEASE-RESISTANT PEPTIDES AS BBB SHUTTLES AND SHUTTLE-CARGO CONSTRUCTS**
AKTIV TRANSPORTIERTE UND PROTEASERESISTENTE PEPTIDE ALS BBB-SHUTTLES UND SHUTTLE-FRACHT-KONSTRUKTE
PEPTIDES RÉSISTANTS À LA PROTÉASE ET TRANSPORTÉS ACTIVEMENT COMME DES NAVETTES BBB ET CONSTRUCTIONS DE NAVETTE-CARGO

(30) Priority: 04.07.2013 EP 13382274
(43) Date of publication of application: 11.05.2016
(73) Proprietor: Universitat de Barcelona, 08028 Barcelona (ES); Fundació Institut de Recerca Biomèdica IRB (Barcelona), 08028 Barcelona (ES)
(72) Inventor: GIRALT LLEDÓ, Ernest, E-08028 Barcelona (ES); TEIXIDÓ TURÀ, Meritxell, E-08028 Barcelona (ES); OLLER SALVIA, Benjamí, E-08028 Barcelona (ES)
(74) Representative: ZBM Patents - Zea, Barlocci & Markvardsen
(86) International application number: PCT/EP2014/064173
(87) International publication number: WO 2015/001015

(56) References cited:
- WO-A1-2011/154887
- WO-A2-00/69900
- US-A1- 2009 318 341
- B. T. BRAZIL: "Model Peptide Studies Demonstrate That Amphipathic Secondary Structures Can Be Recognized by the Chaperonin GroEL (cpn60)", JOURNAL OF BIOLOGICAL CHEMISTRY, vol. 272, no. 8, 21 February 1997 (1997-02-21), pages 5105-5111, XP055088019, ISSN: 0021-9258, DOI: 10.1074/jbc.272.8.5105
- BENJAMÍ OLLER-SALVIA ET AL: "From venoms to BBB shuttles. Synthesis and blood-brain barrier transport assessment of apamin and a non-toxic analog", BIOPOLYMERS, 26 November 2013 (2013-11-26), pages n/a-n/a, XP055088029, ISSN: 0006-3525, DOI: 10.1002/bip.22257
- Wesley L Cosand ET AL: "Concept of internal structural controls for evaluation of inactive synthetic peptide analogs: Synthesis of [Orn-13,14] apamin and its guanidiantion to apamin derivative with full neurotoxic activity", Biochemistry, 1 July 1977 (1977-07-01), pages 2771-2775, XP055088129, Retrieved from the Internet: URL:http://www.pnas.org/content/74/7/2771. full.pdf [retrieved on 2013-11-13]
- None

### SEQUENCE LISTING

<110> Universitat de Barcelona
   Fundació Institut de Recerca Biomèdica (IRB Barcelona)
<120> Actively transported and protease-resistant peptides as BBB shuttles and shuttle-cargo constructs
<130> WO-AVCRI220E
<150> EP13382274.2
   <151> 2013-07-04
<160> 20
<170> PatentIn version 3.5
<210> 1
   <211> 12
   <212> PRT
   <213> Artificial
<220>
   <223> BBB shuttle
<220>
   <221> MISC_FEATURE
   <222> (1)..(1)
   <223> Xaa is an amino acid selected form C, D, E, K, O (Ornithine), Dap (diaminopropionic acid), Dab (diaminobutyric acid), Sec (selenocystein), Pen (penicillamine), AlGly (allyl Glycine) and alpha-Me-alpha-alkGly (alpha-methyl, alpha-alkenylglycine)
<220>
   <221> MISC_FEATURE
   <222> (9)..(9)
   <223> Xaa is an amino acid selected form C, D, E, K, O (Ornithine), Dap (diaminopropionic acid), Dab (diaminobutyric acid), Sec (selenocystein), Pen (penicillamine), AlGly (allyl Glycine) and alpha-Me-alpha-alkGly (alpha-methyl, alpha-alkenylglycine)
<220>
   <221> MISC_FEATURE
   <222> (10)..(10)
   <223> Alanine (A) amino acid can be repeated n times, wherein n is an integer independently selected from 0 and 1
<220>
   <221> MISC_FEATURE
   <222> (11)..(11)
   <223> Alanine (A) amino acid can be repeated n times, wherein n is an integer independently selected from 0 and 1
<220>
   <221> MISC_FEATURE
   <222> (12)..(12)
   <223> Alanine (A) amino acid can be repeated n times, wherein n is an integer independently selected from 0 and 1
<400> 1
   Xaa Lys Ala Pro Glu Thr Ala Leu Xaa Ala Ala Ala
   1               5                   10
<210> 2
   <211> 12
   <212> PRT
   <213> Artificial
<220>
   <223> BBB shuttle
<220>
   <221> MISC_FEATURE
   <222> (10)..(10)
   <223> Alanine (A) amino acid can be repeated n times, wherein n is an integer independently selected from 0 and 1
<220>
   <221> MISC_FEATURE
   <222> (11)..(11)
   <223> Alanine (A) amino acid can be repeated n times, wherein n is an integer independently selected from 0 and 1
<220>
   <221> MISC_FEATURE
   <222> (12)..(12)
   <223> Alanine (A) amino acid can be repeated n times, wherein n is an integer independently selected from 0 and 1
<400> 2
   Cys Lys Ala Pro Glu Thr Ala Leu Cys Ala Ala Ala
   1               5                   10
<210> 3
   <211> 11
   <212> PRT
   <213> Artificial
<220>
   <223> BBB shuttle
<220>
   <221> MISC_FEATURE
   <222> (10)..(10)
   <223> Alanine (A) amino acid can be repeated n times, wherein n is an integer independently selected from 0 and 1
<220>
   <221> MISC_FEATURE
   <222> (11)..(11)
   <223> Alanine (A) amino acid can be repeated n times, wherein n is an integer independently selected from 0 and 1
<400> 3
   Cys Lys Ala Pro Glu Thr Ala Leu Cys Ala Ala
   1               5                   10
<210> 4
   <211> 9
   <212> PRT
   <213> Artificial
<220>
   <223> BBB shuttle
<400> 4
   Cys Lys Ala Pro Glu Thr Ala Leu Cys
   1               5
<210> 5
   <211> 10
   <212> PRT
   <213> Artificial
<220>
   <223> BBB shuttle
<400> 5
   Cys Lys Ala Pro Glu Thr Ala Leu Cys Ala
   1 5 10
<210> 6
   <211> 11
   <212> PRT
   <213> Artificial
<220>
   <223> BBB shuttle
<400> 6
   Cys Lys Ala Pro Glu Thr Ala Leu Cys Ala Ala
   1 5 10
<210> 7
   <211> 12
   <212> PRT
   <213> Artificial
<220>
   <223> BBB shuttle
<400> 7
   Cys Lys Ala Pro Glu Thr Ala Leu Cys Ala Ala Ala
   1 5 10
<210> 8
   <211> 16
   <212> PRT
   <213> Artificial
<220>
   <223> BBB shuttle
<220>
   <221> MISC_FEATURE
   <222> (1)..(1)
   <223> Xaa is an amino acid selected from C, Sec (selenocystein), Pen (penicillamine), Dab (diaminobutyric acid), Dap (diaminopropionic acid), K, O (Ornithine), D, E, AlGly (allyl Glycine) and alpha-Me-alpha-alkGly (alpha-methyl, alpha-alkenylglycine)
<220>
   <221> MISC_FEATURE
   <222> (2)..(2)
   <223> Xaa is an amino acid selected from the group consisting of H, N, L, A, G, F, Y, W, I, V, P, S, T, D, E, K, and M.
<220>
   <221> MISC_FEATURE
   <222> (15)..(15)
   <223> Xaa is an amino acid selected from C, Sec (selenocystein), Pen (penicillamine), Dab (diaminobutyric acid), Dap (diaminopropionic acid), K, O (Ornithine), D, E, AlGly (allyl Glycine) and alpha-Me-alpha-alkGly (alpha-methyl, alpha-alkenylglycine).
<220>
   <221> MISC_FEATURE
   <222> (16)..(16)
   <223> Xaa is an amino acid selected from the group consisting in hystidine (H) , G,F Y, W, I, V, P, N, L, A, S, T, D, E, K and M.
<400> 8
   Xaa Xaa Cys Lys Ala Pro Glu Thr Ala Leu Cys Ala Ala Ala Xaa Xaa
   1               5                   10                  15
<210> 9
   <211> 16
   <212> PRT
   <213> Artificial
<220>
   <223> BBB shuttle
<400> 9
   Cys Asn Cys Lys Ala Pro Glu Thr Ala Leu Cys Ala Ala Ala Cys His
   1               5                   10                  15
<210> 10
   <211> 7
   <212> PRT
   <213> Artificial
<220>
   <223> BBB shuttle
<400> 10
   Lys Ala Pro Glu Thr Ala Leu
   1               5
<210> 11
   <211> 5
   <212> PRT
   <213> Artificial
<220>
   <223> Drug
<400> 11
   Leu Pro Phe Phe Asp
   1               5
<210> 12
   <211> 9
   <212> PRT
   <213> Artificial
<220>
   <223> BBB shuttle
<220>
   <221> MISC_FEATURE
   <222> (1)..(1)
   <223> Xaa is the amino acid 2,3-diaminopropionic acid (Dap)
<220>
   <221> MISC_FEATURE
   <222> (1)..(1)
   <223> Xaa is the amino acid diaminopropionic acid (Dap)
<400> 12
   Xaa Lys Ala Pro Glu Thr Ala Leu Asp
   1               5
<210> 13
   <211> 22
   <212> PRT
   <213> Artificial
<220>
   <223> construct
<400> 13
   Leu Pro Phe Phe Asp Gly Cys Asn Cys Lys Ala Pro Glu Thr Ala Leu
   1               5                   10                  15
   Cys Ala Ala Ala Cys His
               20
<210> 14
   <211> 22
   <212> PRT
   <213> Artificial
<220>
   <223> construct
<400> 14
   Cys Asn Cys Lys Ala Pro Glu Thr Ala Leu Cys Ala Ala Ala Cys His
   1               5                   10                  15
   Gly Leu Pro Phe Phe Asp
               20
<210> 15
   <211> 15
   <212> PRT
   <213> Artificial
<220>
   <223> construct
<400> 15
   Leu Pro Phe Phe Asp Gly Cys Lys Ala Pro Glu Thr Ala Leu Cys
   1 5 10 15
<210> 16
   <211> 18
   <212> PRT
   <213> Apis mellifera
<400> 16
   Cys Asn Cys Lys Ala Pro Glu Thr Ala Leu Cys Ala Arg Arg Cys Gln
   1 5 10 15
   Gln His
<210> 17
   <211> 16
   <212> PRT
   <213> Artificial Sequence
<220>
   <223> BBB shuttle
<220>
   <221> MISC_FEATURE
   <222> (2)..(2)
   <223> Xaa is an amino acid selected from the group consisting of H, N, L, A, G, F, Y, W, I, V, P, S, T, D, E, K, and M.
<220>
   <221> MISC_FEATURE
   <222> (16)..(16)
   <223> Xaa is an amino acid selected from the group consisting of H, G,F, Y, W, I, V, P, N, L, A, S, T, D, E, K and M
<400> 17
   Cys Xaa Cys Lys Ala Pro Glu Thr Ala Leu Cys Ala Ala Ala Cys Xaa
   1 5 10 15
<210> 18
   <211> 10
   <212> PRT
   <213> Artificial Sequence
<220>
   <223> BBB shuttle
<220>
   <221> MISC_FEATURE
   <222> (2)..(2)
   <223> Xaa is the amino acid diaminopropionic acid (Dap)
<400> 18
   Cys Xaa Lys Ala Pro Glu Thr Ala Leu Asp
   1 5 10
<210> 19
   <211> 18
   <212> PRT
   <213> Artificial Sequence
<220>
   <223> construct
<220>
   <221> MISC_FEATURE
   <222> (1)..(1)
   <223> Xaa is the amino acid diaminopropionic acid (Dap)
<400> 19
   Xaa Lys Ala Pro Glu Thr Ala Leu Asp Lys Gln Ile Glu Ile Lys Lys
   1               5                   10                  15
   Phe Lys
<210> 20
   <211> 9
   <212> PRT
   <213> Artificial Sequence
<220>
   <223> drug
<400> 20
   Lys Gln Ile Glu Ile Lys Lys Phe Lys
   1               5

## Description

The present invention relates to the fields of medicine, research and diagnostics, and more specifically to novel peptidic compounds that act as shuttles through the blood-brain barrier (BBB) for the delivery of substances that cannot cross the BBB by themselves. It also refers to shuttle-cargo constructs and their use in therapy or diagnostics.

### BACKGROUND ART

One of the most interesting targets for new medicines, from a pharmaceutical point of view, are among others the ones related to the CNS such as Parkinson, Alzheimer, brain tumours or disease candidate to protein replacement therapy as they are every day more common in developed countries where the longer life expectancy implies more incidence of degenerative diseases. Overcoming the difficulty of delivering therapeutic agents to specific regions of the brain presents a major challenge to treatment of these brain disorders. In its neuroprotective role, the blood-brain barrier (BBB) functions to hinder the arrival of many compounds to the brain.

The BBB is a dynamic interface that separates the brain from the circulatory system and protects the central nervous system (CNS) from potentially harmful chemicals while regulating transport of essential molecules and maintaining a stable environment. It is formed by highly specialized endothelial cells that line brain capillaries and transduce signals from the vascular system and from the brain. These cells restrict the diffusion of microscopic objects (e.g., bacteria) and large or hydrophilic molecules into the brain, while allowing the diffusion of small hydrophobic molecules (O₂, CO₂, hormones). Cells of the barrier actively transport metabolic products such as glucose across the barrier with specific carriers.

The structure and function of the BBB is dependent upon the complex interplay between the different cell types (such as the endothelial cells, astrocytes, and pericytes), and the extracellular matrix of the brain and blood flow in the capillaries.

Certain small molecule drugs may cross the BBB via lipid-mediated free diffusion, most of which have a molecular weight below 400 uma, a log p lower than 5, no more than 3 hydrogen bond donors and less than 7 hydrogen bond acceptors. These chemical properties are lacking in the majority of small molecule drugs, and in all the large molecule drugs. Therapeutic molecules, for instance and antibodies, that might otherwise be very useful in diagnosis and/or highly effective as therapy do not cross the BBB in adequate amounts and so new forms of crossing it are extremely demanded by the pharmaceutical industry.

There are different strategies for BBB crossing and the most promising regarding the transport of large molecules are the ones based on molecular Trojan Horses (MTH) or BBB-shuttles. An MTH is an endogenous peptide, or a peptidomimetic monoclonal (mAb), which traverses the BBB on a specific receptor-mediated transport (RMT) system. Molecules as insulin or transferrin *per se,* are not viable as MTH, since insulin causes hypoglycaemia and transferrin has a very high concentration in plasma. Alternatively, RMT-specific MTHs may be peptidomimetic mAbs that bind exofacial epitopes on the BBB receptor, which are spatially removed from the endogenous ligand binding site. Once bound to the RMT system on the BBB, the MTH undergoes RMT across the BBB. A BBB-shuttle is a peptide able to cross the BBB with a cargo, which cannot cross the BBB by itself. Unfortunately, most of the BBB-shuttle peptides show a low half-life time in human serum, which limit their applications.

On the other hand, to date, only a small number of known venom components are suspected to penetrate the BBB without causing inflammation. Among the few peptidic animal toxins that have been reported to reach the brain after intravenous injection, chlorotoxin and apamin are the most widely known. The former is a 36-mer scorpion peptide in phase II clinical studies for the treatment of glioma. It has been shown to internalize in endothelial cells in vitro and to reach the brain parenchyma in vivo. The latter is an 18-mer peptide from bee venom that accumulates in significant amounts in the brain and spinal cord.

Apamin accounts for 2% of bee (Apis mellifera) venom dry weight and was one of the first K+ channel blockers to be described. Although biodistribution studies have been performed in mice, the permeability of BBB to apamin is controversial. While the accumulation of this peptide in the brain and spinal cord after intravenous injection has been demonstrated, the selectivity for these organs is not well established. The most important residues of apamin are the 4 Cysteine residues (forming 2 disulphide bonds important for the structure, but apparently not too relevant for transport across the BBB) and 2 Arginine residues responsible for its toxicity (cf. C. Devaux et al., European Journal of Biochemistry, vol. 231, pp. 544-550).

Modifications of apamin to reduce toxicity are referred by some authors such as Vincent et al. (cf. Biochemistry, 1975, vol. 14, pp.2521-2525), which describe chemical modifications of several residues, concluding that the most effective on the toxicity are Arg13 and Arg14. Another paper by L.W. Cosand et al. (cf. Proc Natl Acad Sci USA, 1977, vol. 74, pp.2772-2775) describes the substitution of these two residues by ornithin ones, which abolishes toxicity. However, this paper does not investigate if ornithin derivative is capable of crossing the BBB, as it might have lost toxicity because it is no longer able to reach its target inside the brain.

US2009318341 discloses that both the 13 and 14 position of apamin can be substituted with alanine.

Finally, B. T: Brazil et al., in Journal of Biological Chemistry, 1997, vol. 72, pp.5105-5111 discloses hybrid peptides wherein the N-terminal part comprises the apamin-derived sequence CNCKAPETALC, wherein the lysine is labelled with fluorescein and the C-terminal part comprises a sequence derived from the protein rhodanase. The fluorescein is joined to the side chain of the lysine. The sequence is used as a model of alpha helical conformation to study the recognition of secondary structures by the Chaperonine GroEL. However, no mention to the transport through the BBB is found in this document.

From what it is known in the art it is derived that designing small molecules that are able to pass efficiently through the BBB is a challenge and one that is unfeasible for most large molecules. Therefore, there is still the need of finding vectors (BBB-shuttles) that can carry these loads across the BBB to achieve drug delivery to the brain.

### SUMMARY OF THE INVENTION

Inventors have found that some specific peptides with a relatively reduced length of their sequences that comprises the fragment KAPETAL have the capacity to cross the BBB and are able to facilitate the transport into the brain of drugs or other substances useful for diagnosis, referred to as "cargos", which cannot cross the BBB by themselves. In particular, the high permeability values obtained together with the evidence of an active transport mechanism make these peptides very promising BBB-shuttles.

Thus, these peptides, which are derivatives of apamin, are not toxic and are able to cross the BBB. Contrarily to what could be expected, the derivatives of the invention do not lose its toxicity because it cannot enter the brain but most probably because the affinity for its target decreases. Moreover, these results show that the residues of apamin implied in toxicity are not required for transport.

The fact that a peptide crosses the BBB does not mean that it can be a shuttle for other peptides and proteins since it can cross the BBB by passive diffusion. The main advantage of the peptides of the invention is the use of an active transport mechanism to cross the BBB, as they allow in this sense to transport to the brain big cargoes which substantially modify the size and fisico-chemical properties of the shuttles (such as proteins, antibodies, etc) and which is of great interest for the development of the most promising new drugs: monoclonal antibodies or other therapeutic recombinant proteins. Furthermore, as they allow great cargo transport, they can be anchored to nanoparticles, which may be used both for therapeutics and imaging techniques for diagnosis.

Another advantage is the high stability at different pH values and temperatures and the high protease stability, mainly because of the cyclic structures provided by the intrapeptide bridges. This desired property that is usually achieved by incorporating expensive D-amino acids, in the peptides of the invention is achieved using L-amino acids. Moreover, these peptides are biocompatible, have lack of toxicity and are easy to be manufactured.

It is disclosed a peptidic compound of formula

R₁-(V)ₖ-P-(W)ₛ-Y

wherein: R₁ is the group attached to the N-terminal of the first amino acid of the sequence P, via the biradical V, and is selected from the group consisting of hydrogen, CH₃C(=O)-, and maleimide; V is a biradical selected from the group consisting of -NH-(CH₂)ᵣ-C(=O)-, -NH-CH-((CH₂)ᵣNH₂)-C(=O)-, -C(=O)-(CH₂)ᵣ-C(=O)-, -S-(CH₂)ᵣ-, -S-(CH₂)ᵣ-C(=O)-, -O-(CH₂)ᵣ-, -S-CH₂-CH(NH₂)-C(=O)-, -O-(CH₂)ᵣC(=O)-, -(CH₂)ᵣC(=O)-, -NH-O-CH₂-C(=O)-NH-(CH₂)ᵣ-CH(NH₂)-C(=O)-, and
-C≡C-(CH₂)ᵣC(=O)-; the biradical V being attached to R₁ and to the N of the sequence P as follows: R₁-NH-(CH₂)ᵣC(=O)-N(H)ₘ-, R₁-NH-CH-((CH₂)ᵣNH₂)-C(=O)-N(H)ₘ, R₁-C(=O)-(CH₂),-C(=O)-N(H)ₘ-, R₁-S-(CH₂)ᵣ-N(H)ₘ-, R₁-S-(CH₂)ᵣC(=O)-N(H)ₘ-, R₁-O-(CH₂)ᵣN(H)ₘ-, R₁-S-CH₂-CH(NH₂)-C(=O)-N(H)ₘ, R₁-O-(CH₂)ᵣC(=O)-N(H)ₘ-, R₁-(CH₂)ᵣ-C(=O)-N(H)ₘ, R₁=N-O-CH₂-C(=O)-NH-(CH₂)ᵣCH(NH₂)-C(=O)-N(H)ₘ; and R₁-C≡C-(CH₂)ᵣC(=O)-(NH)ₘ P is a biradical of a peptide having 9-20 amino acids residues in length having at least an intrapeptide bond selected from the group consisting of a disulfide bond, an amide bond; a thioether bond, a Se-Se bond, a C-C bond, and a C=C bond; and comprising the amino acid sequence
Xₐₐ¹KAPETALXₐₐ²(A)ₙ₁(A)ₙ₂(A)ₙ₃ (SEQ ID NO:1)
Xaa¹ and Xaa² are an amino acid independently selected form C, D, E, K, O, Dap, Dab, Sec, Pen, AlGly, and alpha-Me-alpha-alkGly; wherein n₁, n₂ and n₃ are integers independently selected from 0 an 1; W is a biradical selected from the group consisting of -NH-(CH₂)ᵣ-C(=O)-, and -NH-CH((CH₂)ᵣNH₂)-C(=O)-; the biradical W being attached to the C=O of the sequence P and to Y as follows: -C(=O)-NH-(CH₂)ᵣ-C(=O)-Y, -C(=O)-NH-CH((CH₂)ᵣNH₂)-C(=O)-Y; Y is the group attached to the C-terminal of the last amino acid of the sequence P, and is selected from the group consisting of -NH₂, -OH, -OR₂ and -NHR₂; R₂ is a radical selected from the group consisting of (C₁-C₆)-alkyl and (CH₂)₂-NH-C(=O)-CH₂-O-NH₂; r is an integer from 1 to 5; k is an integer from 0 to 1; m is an integer from 0 to 1; s is an integer from 0 to 1; with the proviso that: when at least one of n₁, n₂ and n₃ is 0, then at least an intrapeptide bond is between Xₐₐ¹ and Xₐₐ²; when the biradical V is -C(=O)(CH₂)ᵣC(=O)-, then R₁ is hydrogen; when the N of the amino acid of the sequence P to which is attached the biradical V is a biradical -NH-, then m is 1 ,and when is a biradical -N-, then m is 0; and when R₁ is maleimide then the biradical V is -(CH₂)ᵣC(=O)-.

A first aspect of the present invention relates to a peptidic compound of formula

R₁-(V)ₖ-P-(W)ₛ-Y (I)

wherein:
R₁ is the group attached to the N-terminal of the first amino acid of the sequence P, via the biradical V, and is selected from the group consisting of hydrogen, CH₃C(=O)-, and maleimide;
V is a biradical selected from the group consisting of -NH-(CH₂)ᵣ-C(=O)-, -NH-CH-((CH₂)ᵣNH₂)-C(=O)-, -C(=O)-(CH₂)ᵣC(=O)-, -S-(CH₂)ᵣ-, -S-(CH₂)ᵣ-C(=O)-, -O-(CH₂)ᵣ-, -S-CH₂-CH(NH₂)-C(=O)-, -O-(CH₂)ᵣC(=O)-, -(CH₂)ᵣC(=O)-, -NH-O-CH₂-C(=O)-NH-(CH₂)ᵣ-CH(NH₂)-C(=O)-, and -C≡C-(CH₂)ᵣC(=O)-;
the biradical V being attached to R₁ and to the N of the sequence P as follows: R₁-NH-(CH₂)ᵣ-C(=O)-N(H)ₘ-, R₁-NH-CH-((CH₂)ᵣNH₂)-C(=O)-N(H)ₘ, R₁-C(=O)-(CH₂)ᵣ-C(=O)-N(H)ₘ-, R₁-S-(CH₂)ᵣ-N(H)ₘ-, R₁-S-(CH₂)ᵣ-C(=O)-N(H)ₘ-, R₁-O-(CH₂)ᵣ-N(H)ₘ-, R₁-S-CH₂-CH(NH₂)-C(=O)-N(H)ₘ, R₁-O-(CH₂)ᵣ-C(=O)-N(H)ₘ-, R₁-(CH₂)ᵣC(=O)-N(H)ₘ, R₁-NH-O-CH₂-C(=O)-NH-(CH₂)ᵣ-CH(NH₂)-C(=O)-N(H)ₘ; and R₁-C≡C-(CH₂)ᵣC(=O)-(NH)ₘ;
P is a biradical of:
   (a) a peptide having 9-20 amino acids residues in length having at least an intrapeptide bond which is a disulfide bond, and comprises an amino acid sequence which is:
      CKAPETALCAAA (SEQ ID NO:7);
      having at least an intrapeptide disulfide bond between cysteines 1 and 9,
      or a peptide having 9-11 amino acids residues in length having at least an intrapeptide bond which is a disulfide bond and consists of an amino acid sequence selected from the group consisting of
      CKAPETALC (SEQ ID NO:4);
      CKAPETALCA (SEQ ID NO:5); and
      CKAPETALCAA (SEQ ID NO:6); having at least an intrapeptide disulfide bond between cysteines 1 and 9; or alternatively,
   (b) P has 16 amino acid residues and comprises the amino acid sequence
      CNCKAPETALCAAACH (SEQ ID NO:9)
      with an intrapeptide disulfide bond between the first and third cysteine which are cysteines 1 and 11, and between the second and the fourth cysteine which are cysteine 3 and 15, or alternatively;
   (c) P comprises the amino acid sequence
      DapKAPETALD (SEQ ID NO:12)
      with an intrapeptide bond between the Dap and D which is an amide bond;
      W is a biradical selected from the group consisting of -NH-(CH₂)ᵣ-C(=O)-, and -NH-CH((CH₂)ᵣNH₂)-C(=O)-;
      the biradical W being attached to the C=O of the sequence P and to Y as follows: -C(=O)-NH-(CH₂)ᵣ-C(=O)-Y, or -C(=O)-NH-CH((CH₂)ᵣNH₂)-C(=O)-Y;
      Y is the group attached to the C-terminal of the last amino acid of the sequence P, and is selected from the group consisting of -NH₂, -OH, -OR₂, and -NHR₂;
      R₂ is a radical selected from the group consisting of (C₁-C₆)-alkyl and (CH₂)₂-NH-C(=O)-CH₂-O-NH₂;
      r is an integer from 1 to 5;
      k is an integer from 0 to 1;
      m is an integer from 0 to 1;
      s is an integer from 0 to 1;
      with the proviso that:
      when the biradical V is -C(=O)(CH₂)ᵣC(=O)-, then R₁ is hydrogen;
      when the N of the amino acid of the sequence P to which is attached the biradical V is a biradical -NH-, then m is 1, and when the N of the amino acid of the sequence P to which is attached the biradical -V- is a biradical -N-, then m is 0; and
      when R₁ is maleimide then the biradical V is -(CH₂)ᵣ-C(=O)-.

The peptidic compounds of formula (I) have appropriate functional groups suitable for covalent attachment of cargos maintaining the original activity of the cargo until it reaches the site of action. Other peptides also with a relatively reduced length of their sequences that comprises the fragment KAPETAL may also be used as they are able to facilitate the transport into the brain of drugs or other substances useful for diagnosis. Thus, a second aspect of the present invention relates to a construct of formula (II) or a pharmaceutically acceptable salt thereof,

(R₁)ₑ-(Z)_{q1}-(V)ₖ-P-(W)ₛ-(Z)_{q2}-(Y)_{f}, (II)

wherein: P is a biradical of a peptide selected from the group consisting of a peptide as defined for the compound of formula (I) and a peptide consisting of the amino acid sequence KAPETAL (SEQ ID NO:10); R₁, Y, W, k, s, are as defined for the compound of formula (I), V is as defined for the compound of formula (I), or alternatively, V is selected from the group consisting of V₁, V₂ and V₃,
V₁ being 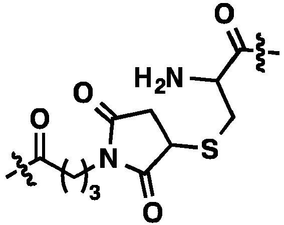
V₂ being 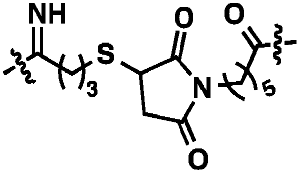
and V₃ being 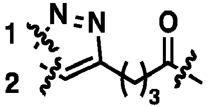 wherein bonds 1 and 2 are both connected to the side-chain of a lysine from the protein or antibody cargo;
   Z is a radical independently selected of a biologically active substance which is an active pharmaceutical ingredient capable of forming an amide bond, an ester bond, a disulfide bond, a thioether bond, an oxime bond, an amine bond, a 1,4-disubstituted 1,2,3-triazole bond (triazole bond), or an hydrazone bond with V or an amide bond or an ester bond with W, and is selected from the group consisting of antiretroviral agents, anticancer agents, antipsychotic agents, antineurodegenerative agents, a therapeutic protein for protein replacement therapy, and antiepileptic agents;or a substance for use in a diagnostic method selected from the group consisting of 5(6)-carboxyfluorescein, biotin, sulforhodamine B, Quantum dots, and superparamagnetic iron oxide nanoparticles (Spion); said substance being substantially unable to cross the BBB by itself;
   q1 and q2 are integers from 0 to 2, being q1 and/or q2 different than 0 and the sum of q1 and q2 being 1 or 2;
   e and f are integers from 0 to 1;
   with the proviso that:
   when q1 is 2 and the biradical V has a non-terminal nitrogen, then one Z is attached to the N-terminal of the first amino acid of the sequence P via the biradical V, and the other Z is attached to the non-terminal nitrogen of the biradical V, and k is 1;
   when q2 is 2 and the biradical W has a non-terminal nitrogen, then one Z is attached to the C=O terminal of the last amino acid of the sequence P, via the biradical W, and the other Z is attached to the non-terminal nitrogen of the biradicals W and s is 1; and
   when q1 is 2 then e is 0, when q2 is 2 then f is 0,
   when q1 is 1 then Z is attached to the N-terminal of the first amino acid of the sequence P, optionally via the biradical V, and e is 0; and
   when q2 is 1 then Z is attached to the C=O terminal of the last amino acid of the sequence P, optionally via the biradical W, and f is 0.
Bonds 1 and 2 of the linker V₃ are both connected to the side-chain of a lysine from the protein or antibody cargo as follows:

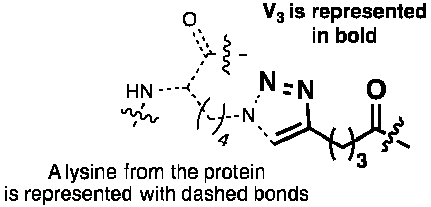

This lysine can be any lysine forming part of the amino acid sequence of the protein. The conjugation technique used comprises transforming the amine of the side (-NH₂) into an azide (-N₃). The azide then reacts with the triple bond of the N-terminal of the peptide forming the 1,2,3-triazol 1,4-disubstituted.

The biradical V₁, V₂ and V₃ are attached to (R₁)ₑ-(Z)_{q1} by the left side of the represented formulae and to the N terminal of the sequence P by the other side of the represented formulae.

The peptidic compounds of formula (I) of the invention, as well as the KAPETAL-NH₂ show several properties that make them useful as BBB-shuttles. It is disclosed the use of a peptide selected from a peptide as above and KAPETAL-NH₂, as a shuttle through the BBB.

Another aspect of the present invention relates to a construct of formula (II) as defined above where Z is a radical of a biologically active substance, for use as a medicament, wherein the biologically active substance is an active pharmaceutical ingredient capable of forming an amide bond, an ester bond, a disulfide bond, a thioether bond, an oxime bond, an amine bond, a 1,4-disubstituted 1,2,3-triazole bond (triazole bond), or an hydrazone bond with V or an amide bond or an ester bond with W, and is selected from the group consisting of antiretroviral agents, anticancer agents, antipsychotic agents, antineurodegenerative agents, a therapeutic protein for protein replacement therapy, and antiepileptic agents.

Another aspect of the present invention relates to a construct of formula (II) as defined above where Z is a superparamagnetic iron oxide nanoparticle or a gadolinium complex, for use as a contrast agent for a magnetic resonance imaging diagnostic method.

Another aspect of the present invention relates to a construct of formula (II) as defined above where Z is selected from the group consisting of 5(6)-carboxyfluorescein, Texas red, rhodamine and quantum dots, for use as a fluorescent prove for an image diagnostic method.

Finally, another aspect of the present invention relates to a pharmaceutical composition or a composition for diagnostic purposes comprising a therapeutically effective amount of the construct as defined above, together with appropriate amounts of pharmaceutically acceptable carriers or excipients and/or acceptable for diagnosis.

### DETAILED DESCRIPTION OF THE INVENTION

Unless otherwise stated, the amino acids cited herein are L-amino acids. The 1-letter code and the 3-letter code have been used indistinctly. For the following amino acids the following abbreviations have been used: diaminopropionic acid (Dap), diaminobutiric acid (Dab), selenocysteine (Sec), penicillamine (Pen), ornithine (O), allyl Glycine (AlGly) and alpha-methyl, alpha-alkenylglycine (alpha-Me-alpha-alkGly). In the context of the present invention penicillamine only embraces D-penicillamine.

The term "intrapeptide bond" as stated herein refers to a bond between the side chains of two amino acids residues.

As mentioned above, it is part of the invention the peptidic compounds of formula R₁-(V)ₖ-P-(W)ₛ-Y (I), wherein: R₁, V, P , W, k and s have the meanings mentioned above.

The amino acid sequence P may range from 9-20 amino acids in length, preferably, between 9-16 amino acids. In particular embodiments may consist of 9, 10, 11, 12 13, 14, 15, and 16 amino acids in length. They comprise at least the contiguous amino acid sequence KAPETAL and an intrapeptide bond. Amino acid modifications that substantially do not affect the transport capacity through the BBB barrier are within the scope of protection. The amino acid sequence P of the invention is formed from amino acids that do not confer general acute toxicity to the peptide. Accordingly, the amino acid sequence P of the invention is at least free of the amino acid R. The amino acid sequence P of the invention may also be free of the amino acid Q.

Some of the amino acids that may be forming the amino acid sequence P together with the KAPETAL fragment are C, N, L, A, M, D, E, K, O,H, G, F, Y, W, I, V, P S, T, Dap, Dab, Sec, Pen, AIGly or alpha-Me-alpha-alkGly. Preferably, the peptidic compounds of the invention are those where n₁, n₂ and n₃ are equal to 1. Also preferably, the peptidic compounds of the invention have one or two intrapeptide bonds. Also preferably, the intrapeptide bonds are amide bonds or disulfide bonds.

Also, preferably the peptidic compounds of the invention have between 9 and 16 amino acids.

Any range given in this document is intended to include the end values of the range.

In a particular embodiment, the peptidic compounds of the invention are those where peptide P comprises the amino acid sequence: DapKAPETALD (SEQ ID NO: 12) with an intrapeptide bond formed between the Dap and D which is an amide bond.

In another particular embodiment, the peptidic compound of formula (I) is that where P is SEQ ID NO: 12, k and s are 0, and R₁ is H and Y is NH₂: H-DapKAPETALD-NH₂.

In another particular embodiment, the peptidic compounds of formula (I) are those where in peptide P, Xₐₐ¹ is Dap and Xₐₐ² is D and the intrapeptide bond is formed between the Dap and D and is an amide bond, the peptide P comprising the amino acid sequence: DapKAPETALD and V is S-CH₂-CH(NH₂)-C(=O), i.e. the amino acid sequence CDapKAPETALD (SEQ ID NO:18).

In another particular embodiment, the peptidic compound of formula (I) is that where P is SEQ ID NO:12, V is S-CH₂-CH(NH₂)-C(=O), k is 1 and s is 0, R₁ is H and Y is NH₂: H-CDapKAPETALD-NH₂.

Peptidic compounds of the invention are those where Xₐₐ₁ and Xₐₐ₂ in peptide P are cysteines and the intrapeptide bond is a disulfide bond between cysteines 1 and 9, the peptide P comprising the amino acid sequence: CKAPETALC(A)n₁(A)n₂(A)n₃ (SEQ ID NO:2), wherein n₁, n₂ and n₃ are integers independently selected from 0 to 1; with the proviso that when n₃ is zero, then the peptide has 9-11 amino acid residues and consists of the sequence: CKAPETALC(A)ₙ₁(A)ₙ₂ (SEQ ID NO:3), being n₁ and n₂ integers independently selected from 0 and 1.

In a particular preferred embodiment, the peptidic compounds of formula (I) of the invention are those mentioned above where P comprises an amino acid sequence selected from the group consisting of: CKAPETALC (SEQ ID NO:4); CKAPETALCA (SEQ ID NO:5); CKAPETALCAA (SEQ ID NO:6); and CKAPETALCAAA (SEQ ID NO:7), having an intrapeptide disulfide bond between the two cysteines of each of the previous amino acid sequences.

The more preferred peptidic compounds of formula (I) are those selected from the following list, where the amino acid sequences have an intrapeptide disulfide bond between the two cysteines:
H-CKAPETALC-NH₂ (SEQ ID NO:4, R₁ is H and Y is NH₂);
H-CKAPETALCA-NH₂ (SEQ ID NO:5, R₁ is H and Y is NH₂);
H-CKAPETALCAA-NH₂ (SEQ ID NO:6, R₁ is H and Y is NH₂); and
H-CKAPETALCAAA-NH₂ (SEQ ID NO:7, R₁ is H and Y is NH₂),

In another preferred embodiment, the peptidic compounds of the invention are those which have 16 amino acid residues and comprise the amino acid sequence CKAPETALCAAA as follows:Xₐₐ³Xₐₐ⁴CKAPETALCAAAXₐₐ⁵Xₐₐ⁶ (SEQ ID NO:8), wherein Xₐₐ³ and Xₐₐ⁵ are independently selected from the group consisting of C, Sec, Pen, Dab, Dap, K, O,D, E, AIGly, and alpha-Me-alpha-alkGly; Xₐₐ⁴ is selected from the group consisting of H, N, L, A, G, F, Y, W, I, V, P, S, T, D, E, K, and M, and Xₐₐ⁶ is selected from the group consisting of H, G,F, Y, W, I, V, P, N, L, A, S, T, D, E, K and M, wherein when Xₐₐ³ and Xₐₐ⁵ are equal to cysteine, then the peptide comprises the amino acid sequence: CXₐₐ⁴CKAPETALCAAACXₐₐ⁶ (SEQ ID NO: 17), with an intrapeptide disulfide bond between the first and third cysteine which are cysteines 1 and 11, and between the second and the fourth cysteine which are cysteine 3 and 15.The inventors have found that the disulfide bond pattern of apamin is not required for BBB transport, which opens the possibility of using peptide shuttles with different disulfide bond connectivity.

In another more preferred embodiment, the peptides of the invention are those mentioned above where Xₐₐ³ and Xₐₐ⁵ are independently selected from the group consisting of C, Sec, Pen, Dab, Dap, K, O, D, and E, Xₐₐ⁴ is selected from the group consisting of N, L, A, and M, and Xₐₐ⁶ is H wherein when Xₐₐ³ and Xₐₐ⁵ are different to cysteine, then the peptide P has at least an intrapeptide disulfide bond between cysteines 3 and 12; and wherein when Xₐₐ³and Xₐₐ⁵ are equal to cysteine, then the peptide comprises the amino acid sequence: CXₐₐ⁴CKAPETALCAAACXₐₐ⁶ (SEQ ID NO: 17), with an intrapeptide disulfide bond between the first and third cysteine which are cysteines 1 and 11, and between the second and the fourth cysteine which are cysteine 3 and 15.

In a more preferred embodiment, the peptidic compound of formula (I) is that where P is CNCKAPETALCAAACH (SEQ ID NO:9), R₁ is H and Y is NH₂: H-CNCKAPETALCAAACH-NH₂, with an intrapeptide disulfide bond between the first and third cysteine and between the second and the fourth cysteine.

In a particular embodiment, the peptidic compound of the invention is that where k is 0, and R₁ is hydrogen. In another particular embodiment, the peptidic compound of the invention is that where s is 0, and Y is NH₂.

In another particular embodiment, the peptidic compounds of the invention are those of formula (I) where k is 0 and s is 0, having the compound the following formula (I₁) being R₁, P, and Y as defined for the compounds of formula (I): R₁-P-Y (I₁).

Preferably, in the any of the above peptidic compounds Y is selected from -NH₂, -OH and -NHR₂. More preferably, Y is -NH₂.

As previously mentioned, the peptides of the invention have proved to be really well transported in the in vitro cell based model of the blood-brain barrier that exhibit a good correlation with the in vivo situation, to have a half-life in serum higher than 24h and to be non-toxic in mice and in zebra fish. As it is illustrated in the Examples, the peptidic compounds of formula (I) as well as the KAPETAL have the capacity to transport substances into the brain, referred as cargos, which cannot cross the BBB by themselves. The use of these compounds as BBB shuttles makes it possible for instance that the research of novel drugs is not only limited to the compounds that can cross the BBB by themselves.

The compounds of formula (I) have appropriate functional groups suitable for covalent attachment of cargos maintaining the original activity of the cargo, until it reaches the site of action. Thus, as mentioned above it is also part of the invention the constructs of formula (II) or their pharmaceutically acceptable salts, which are referred as BBB shuttle-cargo constructs,

(R₁)ₑ-(Z)_{q1}-(V)ₖ-P-(W)ₛ-(Z)_{q2}-(Y)_{f}, (II)

wherein: P is a biradical of a peptide selected from the group consisting of a peptide as defined above, R₁, V, W, Y, k, and s are as defined above, preferably, V is as defined for the compound of formula (I), Z is a radical independently selected of a biologically active substance which is an active pharmaceutical ingredient capable of forming an amide bond, an ester bond, a disulfide bond, a thioether bond, an oxime bond, an amine bond, a 1,4-disubstituted 1,2,3-triazole bond (triazole bond), or an hydrazone bond with V or an amide bond or an ester bond with W, and is selected from the group consisting of antiretroviral agents, anticancer agents, antipsychotic agents, antineurodegenerative agents, a therapeutic protein for protein replacement therapy, and antiepileptic agents; or a substance for use in a diagnostic method selected from the group consisting of 5(6)-carboxyfluorescein, biotin, sulforhodamine B, Quantum dots, and superparamagnetic iron oxide nanoparticles (Spion); said substance being substantially unable to cross the BBB by itself;
q1 and q2 are integers from 0 to 2, being q1 and/or q2 different than 0 and the sum of q1 and q2 being 1 or 2;
e and f are integers from 0 to 1;
with the proviso that:
   when q1 is 2 and the biradical V has a non-terminal nitrogen, then one Z is attached to the N-terminal of the first amino acid of the sequence P via the biradical V, and the other Z is attached to the non-terminal nitrogen of the biradical V, and k is 1;
   when q2 is 2 and the biradical W has a non-terminal nitrogen, then one Z is attached to the C=O terminal of the last amino acid of the sequence P, via the biradical W, and the other Z is attached to the non-terminal nitrogen of the biradicals W and s is 1; and
   when q1 is 2 then e is 0, when q2 is 2 then f is 0,
   when q1 is 1 then Z is attached to the N-terminal of the first amino acid of the sequence P, optionally via the biradical V, and e is 0; and
   when q2 is 1 then Z is attached to the C=O terminal of the last amino acid of the sequence P, optionally via the biradical W, and f is 0.

The constructs of formula (II) where the biradical P is a peptide consisting of the amino acid sequence KAPETAL(SEQ ID NO:10) and the rest of the values of the constructs of formula (II) being as defined above are also part of the invention.

The term "substantially unable to cross the BBB" means that it is not capable of crossing the BBB, or if it does is in an amount that is therapeutically non-effective.

The substances from which radical Z is derived include a wide range of substances having pharmacological or diagnostic utility. These substances can be active pharmaceutical ingredients, in particular, antiretroviral agents, anticancer agents, antipsychotic agents, antineurodegenerative agents, antiepileptic drugs or replacement therapy agents. In a preferred embodiment, Z is a radical derived from an active pharmaceutical ingredient capable of forming an amide bond, an ester bond, a disulfide bond, a thioether bond, an oxime bond, an amine bond, a 1,4-disubstituted 1,2,3-triazole bond (triazole bond) or a hydrazone bond with V or an amide bond or an ester bond with W, the mentioned active ingredient being unable to cross the BBB by themselves.

An Example of an active pharmaceutical ingredient is dopamine. Dopamine is a key neurotransmitter in the central nervous system; in particular, striatal dopamine depletion is associated with clinical conditions of Parkinsonism. Dopamine shown interesting properties as a drug but cannot cross the BBB. In particular, as illustrated in the examples, the constructs of formula (II) allow dopamine to be useful as a drug without having the undesired side effects of the actual therapy, and being a simpler and none invasive technique compared with the existing ones.

In a preferred embodiment, the constructs of the invention are those wherein the pharmaceutical active agent is L-dopamine.

Other possible cargos are, for example, peptides, proteins, polymers and antibodies, which have applications as therapeutic or diagnosis agents but that are unable to cross the BBB properly. In a particular embodiment, the peptide is the peptide LPFFD (SEQ ID NO:11). In another particular embodiment, the peptide is the peptide KQIEIKKFK (SEQ ID NO:20).

In a particular embodiment, antibodies are used as cargos. In a preferred embodiment the antibody is an antibody useful for the treatment of cancer. Examples of antibodies that can be used are cetuximab and bevacizumab.

In another particular embodiment proteins are used as cargoes. In a preferred embodiment the protein is a therapeutic protein for protein replacement therapy.

Substances derived from Z also include other substances that can be interesting to transport to the brain but they do not do it properly by themselves, for example contrast agents for magnetic resonance imaging (MRI). Among clinical devices used for clinical cancer diagnosis, MRI outstands as non-invasive and non-destructive powerful imaging modality that provides internal images of living organisms with no limits in the depth of analysis and with a resolution of 10 to 100 microns. It is a valuable technique widely used in cancer diagnosis and research. For early detection of cancer (as well as other diseases) is interesting the use of contrast agents, which can be selectively directed to specific markers located in certain tissues, creating a small accumulation of contrast agent in this tissue. This small accumulation of contrast agent is enough to detect unequivocally the existence, for example, of a tumor in very early states. Contrast agents can also be used as diagnostic methods for other diseases of the central nervous system such as Alzheimer's disease, or as a tool for pre-operative MRI scan that will guide the surgeon. In all these cases the contrast agent used needs to cross the BBB. Examples of typical contrast agents include chelates of paramagnetic elements such as gadolinium or manganese (effect on T1), or the use of superparamagnetic nanoparticles of iron oxide (SPION nanoparticles) (T2 effect). Iron oxide nanoparticles are iron oxide particles with diameters between about 1 and 100 nanometers. The two main forms are magnetite (Fe₃O₄) and its oxidized form maghemite (γ-Fe₂O₃). They have attracted extensive interest due to their superparamagnetic properties and their potential applications in many fields (although Cu, Co and Ni are also highly magnetic materials, they are toxic and easily oxidized). Superparamagnetism is a form of magnetism, which appears in small ferromagnetic or ferrimagnetic micro or nanoparticles, every particle consisting of one magnetic domain. A magnetic domain is a region within a magnetic material which has uniform magnetization. This means that the individual magnetic moments of the atoms are aligned with one another and point in the same direction.

Other non-MRI diagnostic agents include fluorescent dyes or probes such as carboxyfluorescein and its derivatives, rhodamine and its derivatives, or the use of quantum dots (nanoparticles formed by semiconductor materials with high fluorescence quantum yield). The latter, combined with the use of two-photon microscopy, like in the MRI technique, allows in vivo imaging of living organisms in a no-invasive manner, thus allow their use as diagnostic tool.

A quantum dot is a portion of matter (e.g., semiconductor) whose excitons are confined in all three spatial dimensions. Consequently, such materials have electronic properties intermediate between those of bulk semiconductors and those of discrete molecules. Quantum dots are semiconductors whose electronic characteristics are closely related to the size and shape of the individual crystal. Generally, the smaller the size of the crystal, the larger the band gap, the greater the difference in energy between the highest valence band and the lowest conduction band becomes, therefore more energy is needed to excite the dot, and concurrently, more energy is released when the crystal returns to its resting state.

Also in a preferred embodiment the cargo Z includes antibodies useful in diagnostic methods. In another preferred embodiment, the constructs of the invention are those where Z is a radical of a compound selected from the group consisting of an antibody, 5(6)-carboxyfluorescein, biotin, sulforhodamine B, Quantum dots, and Spion. Other possible cargos are gold nanoparticles.

Preferred values of P for the compound of formula (I) are also preferred values for the construct of formula (II), optionally in combination with any other embodiment mentioned above or below.

In a particular embodiment, the constructs of the invention are those where the peptide P has at least an intrapeptide disulfide bond between cysteines, and comprises the amino acid sequence CKAPETALC(A)n₁(A)n₂(A)n₃ (SEQ ID NO:2), n₁, n₂ and n₃ are integers independently selected from 0 and 1; with the proviso that when at least one of n₁, n₂ and n₃ is 0, then a intrapeptide bond is between the two cysteine of the SEQ ID NO:2.

In another particular embodiment, the constructs of the invention are those where the peptide P has at least an intrapeptide disulfide bond between cysteines, and comprises the amino acid sequence CKAPETALC(A)n₁(A)n₂(A)n₃ (SEQ ID NO:2), n₁, n₂ and n₃ are integers independently selected from 0 an 1; with the proviso that when at least one of n₁, n₂ and n₃ is 0, then the peptide has 9-11 amino acid residues and consists of the sequence CKAPETALC(A)ₙ₁(A)ₙ₂ (SEQ ID NO:3) being n₁ and n₂ integers independently selected from 0 and 1.

In a preferred embodiment, the constructs of formula (II) are those which have the formula (II₁) wherein: V, P, and k are as defined above, and Z is a radical of a biologically active substance or a substance for use in a diagnostic method, said substance being substantially unable to cross the BBB by itself: Z-((V)ₖ-P-NH₂ (II₁).

In a more preferred embodiment, the constructs of the invention are those, where the fragment P-Y is selected from the group consisting of:
a biradical of CKAPETALC-NH₂ (SEQ ID NO:4 and Y being NH₂);
a biradical of CKAPETALCA-NH₂ (SEQ ID NO:5 and Y being NH₂);
a biradical of CKAPETALCAA-NH₂ (SEQ ID NO:6 and Y being NH₂);
a biradical of CKAPETALCAAA-NH₂ (SEQ ID NO:7 and Y being NH₂); and
a biradical of CNCKAPETALCAAACH-NH₂ (SEQ ID NO:9 and Y being -NH₂).

The most preferred constructs are those selected from the group consisting of:
L-Dopa-CNCKAPETALCAAACH-NH₂;
L-Dopa-CKAPETALC-NH₂;
H-LPFFDGCNCKAPETALCAAACH-NH₂ (R₁ is H, Z is SEQ ID NO:11, V is G, P is SEQ ID NO:9, Y is NH₂, being the amino acidic fragment LPFFDGCNCKAPETALCAAACH (SEQ ID NO:13));
H-CNCKAPETALCAAACHGLPFFD-NH₂ (R₁ is H, P is SEQ ID NO:9, W is G, Z is SEQ ID NO:11, Y is NH₂, being the amino acidic fragment CNCKAPETALCAAACHGLPFFD (SEQ ID NO:14));
H-LPFFDGCKAPETALC-NH₂ (R₁ is H, Z is SEQ ID NO:11, V is G, P is SEQ ID NO:4, Y is NH₂, being the amino acidic fragment LPFFDGCNCKAPETALCAAACH (SEQ ID NO:15));
5(6)-carboxifluorescein-CNCKAPETALCAAACH-NH₂;
Biotin-CNCKAPETALCAAACH-NH₂;
Sulforhodamine B-GCNCKAPETALCAAACH-NH₂;
Sulforhodamine B-GCKAPETALC-NH₂;
Lysozyme-V₃-CNCKAPETALCAAACH-NH₂.

Other constructs that are between the most preferred ones are those selected from the group consisting of:
L-Dopa-(Dap)KAPETAL-NH₂;
H-(Dap)KAPETALDKQIEIKKFK-NH₂;
Cetuximab-V₃-CNCKAPETALCAAACH-NH₂;
Cetuximab-V₂-DapKAPETALD-NH₂;
Quantum dot-PEG-NH-V₁-DapKAPETALD-NH₂; and
Gold nanoparticle-CDapKAPETALD-NH₂;
   V₁ being 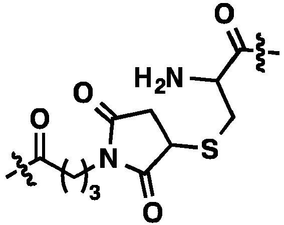
   V₂ being
   and V₃ being
wherein bonds 1 and 2 are both connected to the side-chain of a lysine from the protein or antibody cargo.

The peptidic compounds of formula (I) and of formula (II), as well as their salts 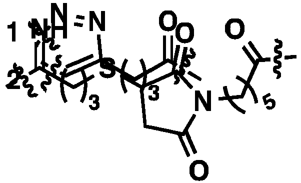 may exist in solvated as well as unsolvated forms, including hydrated forms. Thus, in their structure may contain stoichiometric amounts of solvent in the case of solvates, or water in the case of hydrates. It should be understood that this invention encompasses all the solvated forms, as well as unsolvated. Obtaining solvates and hydrates depends on the solvent used and the crystallization conditions that can be determined by the person skilled in the art.

As mentioned above according to the cargo of the constructs of formula (II), they will be useful in therapy or diagnosis.

The constructs of formula (II) as defined above wherein Z is a radical of a biologically active substance being substantially unable to cross the BBB by itself, for use as a medicament form part of the invention. Preferably, the substances with biological activity are pharmaceutical active ingredients, in particular selected from the group of active ingredients mentioned above.

It is also part of the invention the constructs of formula (II) where Z is a radical of dopamine for use in the treatment of Parkinson's disease. This can also be formulated as the use of constructs of formula (II) where Z is a radical of dopamine in the preparation of a medicament for the treatment of Parkinson's disease. Therefore, this aspect is related to a method of treatment and/or prophylaxis of a mammal, including a human, suffering from or being susceptible to develop Parkinson's disease, this method comprise the administration of a therapeutically effective amount of a compound of formula (II) with Z being a radical from dopamine, together with pharmaceutically acceptable excipients or carriers.

It also form part of the invention the constructs of formula (II) where Z is a peptide radical of the peptide LPFFD (SEQ ID NO:11) for use in treatment of Alzheimer's disease The use of peptide LPFFD (SEQ ID NO:9) for the treatment of Alzheimer's disease described in C. Soto et al., Nature Medicine 1998, vol.4, p.822-826. This aspect can also be formulated as the use of constructs of formula (II) where Z is a peptide radical of the LPFFD (SEQ ID NO:11) for the preparation of a medicament for the treatment of Alzheimer's disease Therefore, this aspect also relates to a method for the treatment and/or prophylaxis of a mammal, including a human, suffering from or being susceptible to develop Alzheimer's disease this method comprise the administration of a therapeutically effective amount of a compound of formula (II) with Z being a peptide radical of the peptide the LPFFD (SEQ ID NO:11) together with pharmaceutically acceptable excipients or carriers.

It also forms part of the invention the constructs of formula (II) where Z is a peptide radical of the peptide KQIEIKKFK (SEQ ID NO:20), a selective hemichannel blocker, for use in treatment of ischemia or epilepsy. This aspect can also be formulated as the use of constructs of formula (II) where Z is a peptide radical of the KQIEIKKFK (SEQ ID NO:20) for the preparation of a medicament to treat treatment of ischemia or epilepsy. Therefore, this aspect also relates to a method of treatment of a mammal, including a human, suffering from ischemia or epilepsy, this method comprise the administration of a therapeutically effective amount of a compound of formula (II) with Z being a peptide radical of the peptide the KQIEIKKFK (SEQ ID NO:20) together with pharmaceutically acceptable excipients or carriers.

It is also part of the invention the constructs of formula (II) where Z is an antibody for use in treatment of cancer. Examples of antibodies for use in the treatment of cancer are bevacizumab or cetuximab.

It is also part of the invention the constructs of formula (II) where Z is a gold nanoparticles for use in photothermal and phtodynamic therapies as well as for drug and gene delivery.

It is also considered part of the invention the constructs of formula (II) where Z is a superparamagnetic iron oxide nanoparticle (SPION nanoparticle), a gadolinium or manganese complex, for the use as contrast agent in a diagnostic method for magnetic resonance imaging. This technique is used for the localization and identification of malignant tumours, metastases of these ones or recurrences. It is also use for the diagnosis of Alzheimer's disease. This aspect can be also formulated as the use of a construct of formula (II) where Z is a superparamagnetic iron oxide nanoparticle (SPION nanoparticle), a gadolinium or manganese complex, for the preparation of a contrast agent for a method of magnetic resonance imaging. Preferably, Z is a superparamagnetic iron oxide nanoparticle (SPION nanoparticle).

The term "contrast agent" refers to agents that enhance the visibility of certain structures that are otherwise difficult to see during the scanning. These agents are used for injection into the vascular system to a local display.

It is also part of the invention the constructs of formula (II) where Z is a radical of a fluorescent compound for use as a fluorescent probe in a method of imaging using two-photon microscopy, obtaining in vivo imaging of living organisms in a non-invasive way, serving as a diagnostic tool. Examples of suitable fluorescent compounds are 5(6)-carboxyfluorescein, Texas red, rhodamine, sulforhodamine or quantum dots. Preferably, the fluorescent compound is 5(6)-carboxyfluorescein or quantum dots.

The peptide compounds of formula (I) and the constructs shuttle-cargo of formula (II) can be generated wholly or partly by chemical synthesis. The amino acids required for the preparation of compounds of formula (I) are commercially available. The compounds of formula (I) and constructs of formula (II) can be prepared easily, for example by synthesis in liquid phase or, preferably, by solid-phase peptide synthesis, for which there are a number of procedures published (cf. M. Amblard, et al., "Methods and protocols of modern solid-phase peptide synthesis. Molecular Biotechnology 2006, Vol. 33, p. 239-254). The compounds of formula (I) or the constructs of formula (II) can also be prepared by any combination of liquid phase synthesis and/or solid phase synthesis. For example, by synthesizing the body of the shuttle through solid-phase synthesis and, subsequently removing protecting groups in solution. The binding of the cargo to the shuttle can be performed in solid phase or in solution. Particular shuttle and the binding to specific cargos are disclosed in more detailed in the examples.

The peptides of the invention can also be obtained by generation of a DNA template and subcloning into an expression vector. (J.H. Lee et al. Eur. J. Biochem. 2001. vol. 268. pp. 2004-2012)

It is also part of the invention the pharmaceutical compositions comprising a therapeutically effective amount of the constructs shuttle-cargo of formula (II) as defined above where Z is a radical of a biologically active substance, together with appropriate amounts of pharmaceutically acceptable diluents or carriers.

The term "therapeutically effective amount" as used herein, refers to the amount of a compound that, when administered, is enough to prevent development of, or alleviate to some extent, one or more symptoms of the disease which is addressed. The particular dose of compound administered according to this invention will of course be determined by the particular circumstances surrounding the case, including the compound administered, the via of administration, the particular condition being treated, and similar considerations.

The term "pharmaceutical composition" refers to a mixture of a compound described herein with other chemical components, such as diluents or carriers. The pharmaceutical composition facilitates administration of the compound to an organism.

The terms "pharmaceutically acceptable excipients or carriers" refers to pharmaceutically acceptable material, composition or vehicle. Each component must be pharmaceutically acceptable in the sense of being compatible with the other ingredients of the pharmaceutical composition. It must be also suitable for use in contact with tissues or organs of humans and animals without excessive toxicity, irritation, allergic response, immunogenicity, or other problems or complications commensurate with a benefit/risk ratio.

A composition which comprises a construct according to the present invention may be administered alone or in combination with other treatments, either simultaneously or sequentially, depending on the condition to be treated.

Finally, also form part of the invention the compositions with diagnostic purposes, which have been called contrast agent or fluorescent probes depending on the construct radical Z of formula (II). These compositions comprise an effective amount of the constructs shuttle-cargo of formula (II) as defined above, where Z is a radical of a substance for use in a diagnostic method for MRI or two-photon spectroscopy, this substance being not able to cross the BBB by itself, together with appropriate amounts of pharmaceutically acceptable excipients or carriers and/or acceptable for diagnosis.

The term "diagnostic acceptable" refers to those excipients or carriers suitable for use in diagnostic technology. They should not adversely affect the stability, safety or efficacy of the composition. Generally, the pharmaceutically acceptable excipients or carriers and/or acceptable for diagnosis are physiologically acceptable sterile medium, i.e. isotonic aqueous solutions.

The compositions of the present invention may be administered in parenteral form suitable for injection, infusion or implantation into the body.

In an additional aspect of the invention a kit comprising the contrast agent according to the present invention in a container together with instructions for use in a diagnostic magnetic resonance imaging is provided.

In another aspect of the invention a kit comprising the fluorescent probe according to the present invention in a container together with instructions for use in a diagnostic method by two-photon microscopy is provided.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

Protected amino acids, handles and resins were supplied by: Luxembourg Industries (Tel-Aviv, Israel), Neosystem (Strasbourg, France), Calbiochem-Novabiochem AG (Laufelfingen, Switzerland), Bachem AG (Bubendorf, Switzerland) or Iris Biotech (Marktredwitz, Germany). Other reagents and solvents used are summarized in Table 1.

**Table 1 Commercials suppliers and reagents used. DCM passed through an Al₂O₃ column. DMF is stored on molecular sieves 4Å and nitrogen is bubbled in order to eliminate volatile agents.**

| Commercial supplier | Reagents and solvents |
|---|---|
| Albatros Chem. Inc. | N-hydroxybenzotriazole (HOBt) |
| Aldrich | L-dopa, piperidine, α-ciano-4-hydroxicinnamic acid (ACH), triisopropylsilane (TIS), (MES), N-Ethyl-N'-(3-dimethylaminopropyl)carbodiimide, 5(6)-carboxyfluorescein, HEPES, lucifer yellow, NaCl, KCl, CaCl₂, MgCl₂, NaHCO₃, glucose, HBSS, dithiothreitol (DTT), trytil chloride, sodium dithioethylcarbamate, 6-maleimidohexanoic acid, 4-maleimidobutyric acid N-succinimidyl ester, lysozyme from chicken egg white, sodium ascorbate, hexynoic acid, 1H-imidazole-1-sulfonyl azide, DMEM, Porc skin gelatin type A and all other reagents unless otherwise specified. |
| Applied GL Biochem Shangai | 1 -hydroxy-7-azabenzotriazole (HOAt) |
| Cell applications | bovine brain endothelial cells |
| Charles river | wistar rats |
| Corning Costar | transwells bovine model (0.33 cm²; pore size 0.4 µm) |
| | transwells human model (1.13 cm², pore size 0.4 µm) 12-well and 24-well cell culture-treated plates, BD matrigel growth factor reduced matrix, 100mm diameter Petri Dish |
| Biochrom A.G. | Gentamycin 10 mg/mL, trypsin-EDTA |
| Innoprot | Endothelial cells medium (ECM) |
| Jescuder | NaOH |
| KaliChemie | trifluoroacetic acid (TFA) |
| Lonza | culture medium |
| Merck | molecular sieves 4Å, Thin layer chromatography layers (TLC), cupper sulfate (CuSO₄) |
| Millipore | polyvinylidine diFluoride (PVDF) filters 0.45 µm |
| Novabiochem | benzotriazol-1-yl-oxytripyrrolidinophosphonium hexafluorophosphate (PyBOP), N-hydroxisuccinimide (NHS), hydroxylamine Novatag™ resin |
| Pierce | Pierce^{(R)} iodination beads |
| Scharlau | dichloromethane (DCM), dimethylformamide (DMF), MeOH, MeOH, tert-butylmethylether |
| SDS | acetone, MeCN, toluene |
| Thermo Scintific | Slide-A-Lyzer MINI dialysis device floats |
| GE-Healthcare | Nap-5 desalting columns, PD-10 desalting columns |
| Iris Biotech | All amino acids except otherwise specified. |
| Acros Organics | sulforhodamine b sulfonyl chloride |
| PCAS BioMatrix Inc. | ChemMatrix® resin |

General considerations about the synthesis. Solid-phase peptide elongation and other solid-phase manipulations were carried out manually in polypropylene syringes fitted with a polyethylene porous disk. Solvents and soluble reagents were removed by suction. Washings between different synthetical steps were carried out with dimethylformamide (DMF) (5 x 0.5 min) and dichloromethane (DCM) (5 x 0.5 min) using 10 mL of solvent/g of resin each time.

Identification tests. The test used for the identification and control of the synthesis was the following: A) Kaiser colorimetric assay for the detection of solid-phase bound primary amines (E. Kaiser et al., Anal. Biochem. 1970, vol. 34, pp. 595-598); B) p-nitro phenyl ester test for secondary amines bound to solid-phase (A. Madder et al., Eur. J. Org. Chem. 1999, pp. 2787-2791).

Protocols used during the synthesis of the peptidic compounds of formula (I) and the constructs of formula (II). The constructs were synthesized at a 100 µmol scale using the following methods and protocols:
Scale of the synthesis. All synthesis were performed in a 100 µmol scale.

Resin initial conditioning. The Rink amide ChemMatrix resin was conditioned by washing with MeOH (5 x 30 s), DMF (5 x 30 s), DCM (5 x 30 s), 1% TFA in DCM (1 x 30 s and 2 x 10 min), DCM (5 × 30 s), DMF (5 × 30 s), DCM (5 x 30 s), 5 % DOEA in DCM (1 x 30 s, 2 x 10 min), DCM (5 x 30 s), DMF (5 x 30 s).

Fmoc group removal. Removal of the 9-fluorenylmethyloxycarbonyl (Fmoc) protecting group was done with 20% (v/v) piperidine in DMF using a treatment of 30 s followed by two treatments of 10 minutes each. Two additional treatments with DBU, toluene, piperidine, DMF (5%, 5%, 20%, 70%) (2 × 5 min) were performed to ensure the removal of the Fmoc group from secondary amines (proline).

### Coupling methods described for 100 µmol scale:

Coupling method 1: The protected amino acid (4 eq., 400 µmol), TBTU (4 eq, 400 µmols, 128 mg) dissolved in DMF (1-3 mL/g resin) were added sequentially to the resin, subsequently DIEA was added (8 eq, 800 µmol, 136 µl). The mixture was allowed to react with intermittent manual stirring for 1 h. The solvent was removed by suction and the resin washed with DMF (5 x 30 s) and DCM (5 x 30 s). The extent of coupling was checked by the Kaiser colorimetric assay. The Fmoc group was removed.
Coupling method 2: The protected amino acid (4 eq. 400 µmols), PyBOP (4 eq, 400 µmols, 208 mg), HOAt (12 eq, 1.2 mmol, 163 mg) dissolved in DMF (1-3 mL/g resin) were added sequentially to the resin, subsequently DIEA was added (12 eq, 1.2 mmol, 204 µL). The mixture was allowed to react with intermittent manual stirring for 1 h. The solvent was removed by suction and the resin washed with DMF (5 x 30 s) and DCM (5 x 30 s). The coupling reaction was carried out twice under the same conditions. The extent of coupling was checked by the p-nitro phenyl ester colorimetric assay. The Fmoc group was removed.

Coupling of the cargo (Z): Depending on the nature of the cargo, it will be linked to the BBB-shuttle in solid-phase or in solution.

Coupling of the cargo in solid-phase: Depending on the nature of the cargo, it will be linked to the BBB-shuttle through different types of chemical bonds.
A) For cargoes with a COOH moiety (e.g. L-dopa, 5(6)-carboxyfluorescein, biotin) the coupling of the cargo onto the BBB-shuttle was done in solid-phase: the BBB-shuttle provided with a NH₂ group was used and was reacted with 4 eq of Cargo-COOH, using 4 eq of PyBOP and 12 eq of HOAt as coupling reagents and 12 eq of DIEA as a base in DMF during 2h. The mixture was allowed to react with intermittent manual stirring. The solvent was removed by suction, and the resin was washed with DMF (5 × 30 s) and DCM (5 × 30 s). The coupling was repeated once. The extent of coupling was checked by the p-nitro phenyl test.
B) For cargoes with a sulfonyl chloride (e.g. sulforhodamine b sulfonyl chloride) the coupling of the cargo onto the BBB-shuttle was done in solid-phase: the BBB-shuttle provided with a NH₂ group was used and was reacted with 2 eq of Cargo-SO₂Cl using 4 eq of DIEA as a base in DMF during 1.5 h at 50 °C. The mixture was allowed to react with intermittent manual stirring. The solvent was removed by suction, and the resin was washed with DMF (5 × 30 s) and DCM (5 × 30 s). The coupling was repeated once overnight.

Final cleavage of the resin and side-chain deprotection: It was carried out by treating resin with TFA (95%), H₂O (2.5%) and TIS (2.5%) (2 h). Tert-butyl ether was added to the obtained product and the mixture was centrifuged (3 x 8 min). The supernatant was discarded and the pellet was resuspended in a mixture of H₂O, MeCN and TFA (1000:1000:1). The product was filtered out and frozen.

### The coupling of protein cargoes:

The linkage of protein cargoes to the BBB-shuttle could be done by one of the following technologies.
Tech 1 - Conjugation by an alkyne-azide cycloaddition: The coupling of lysozyme through lysine residues was performed in solution using the protocol described in S.F.M. van Dongen et al.; Bioconjugate Chem. 2009, vol. 20, pp. 20-23 with some modifications. Briefly, to 25 µL of cargo solution (3.3·10⁻⁹ mol) an aqueous solution of K₂CO₃ (10 µL, 2mg/mL) was addded along with 0.7 µL of Cu₂SO₄·H₂O. After mixing, a solution of imidazole-1-sulfonyl azide hydrochloride in milliQ water was added (2.7 µL, 25 mg/mL, 100 eq) and the reaction was strired overnight. The protein buffer was exchanged by PBS using a PD10 G25 desalting columng. To this solution 20 µL of 5 mM peptide were added. 450 µL of the resulting solution is mixed with 20 µL of hexynoic acid-peptide, 33.75 µL of 12.5 µL CuSO₄ premixed with 25.0 µL of ligand (Tris(3-hydroxypropyltriazolylmethyl)amine), 112.5 µL of aminoguanidine, 112.5 µL of sodium ascorbate, 171.25 µL of PBS.
Tech 2. Reduction-Alkylation of interchain cargo disulfides
   2.1. Limited disulfide reduction with DTT: The cargo solution was diluted (2 mgmL⁻¹, 500 µL) and was partially reduced by DTT (3.25 eq) in 0.025 M sodium borate pH 8, 0.025 M NaCl, 1 mM EDTA for 2 h at 37 °C. The excess of DTT was purified away from the partially reduced cargo by size exclusion (Nap 5, GE Healthcare following manufacturer instructions). The concentration of cargo-cysteine thiols produced was determined by titrating with DTNB, typically resulting in 3.0 to 4.0 thiols/ cargo. The conjugate is produced by alkylation of free thiols with the malimide-bearing peptide.
   2.2. Limited disulfide reduction with TCEP: The cargo solution was diluted (2 mgmL⁻¹, 500 µL) and was partially reduced by TCEP (2.5 eq respectively) in 0.025 M sodium borate pH 8, 0.025 M NaCl, 1 mM EDTA for 2 h at 37 °C. No purification was needed. The concentration of cargo -cysteine thiols produced was determined by titrating with DTNB, typically resulting in 3.0 to 4.0 thiols/ cargo. The conjugate is produced by alkylation of free thiols with the malimide-bearing peptide.
   2.3. Full reduction followed by partial reoxidation: This reaction is performed with DTNB after total reduction with DTT: The cargo solution was diluted (2 mgmL⁻¹, 500 µL) and was totally reduced by DTT (150 eq) in 0.025 M sodium borate pH 8, 0.025 M NaCl, 1 mM EDTA for 2 h at 37 °C. The excess of DTT was purified away from the partially reduced cargo by size exclusion (Nap 5, GE Healthcare following manufacturer instructions). The fully reduced cargo was cooled to 0 °C and then treated with 2.0 equivalents of DTNB (0°C, 20 min). Without further purification the cargo was alkylated by following the protocol above (alkylation 1).
      Alkylation: Partially reduced cargo was alkylated with 1.1 molar equiv of 5(6)-carboxifluorescein-L-Lys(maleimido)-BBB shuttle-NH₂ or rhodamine b-L-Lys(maleimido)-BBB shuttle-NH₂/thiol. The alkylation reaction was performed at 0 °C for 30 min. Cysteine (1 mM final) was used to quench any unreacted, excess of 5(6)-carboxifluorescein-L-Lys(maleimido)-BBB shuttle-NH₂ or rhodamine b-L-Lys(maleimido)-BBB shuttle-NH₂ The excess of peptide was removed from the partially reduced cargo by size exclusion (Nap 5, GE Healthcare following manufacturer instructions). The cargo concentration was quantified using Nanodrop.
Tech 3 - Conjugation at the N-termini: 25 µL of cargo solution (3.3·10⁻⁹ mol) was diluted with 75 µL of phosphate buffer 25 mM pH 6.5. Pyridoxal 5'-phosphate (PLP) (298 eq, 1.0·10⁻⁷ mol, 2.5·10⁻⁵ g) was dissolved in 100 µL of phosphate buffer 25 mM and the pH was made up to 6.5 with 1 M HCl. The cargo solution was mixed with the PLP solution and incubated at 20 °C for 48 h. A Nap-5 size exclusion column was used to separate the excess small molecules from the derivatized cargo using phosphate buffer 25 mM pH 6.2. The cargo was concentrated to 100 µL using a Vivaspin 500 with 50 kDa MWCO and washed twice with phosphate buffer 25 mM pH 6.2. The BBB shuttle peptide bearing an aminooxy moiety (60 eq, 2.0·10⁻⁷ mol) was dissolved in 50 µL of phosphate buffer 25 mM and the pH was made up to 6.2 with 1 M aqueous HCl. The transaminated cargo from the Nap-5 column were mixed with the peptide solution and with 50 µL of a 4·10⁻³ M solution of aniline (60 eq, 2.0·10⁻⁷ mol, 1.9·10⁻⁵ g); the mixture was left to react for 48 h. Excess reagents were removed using a Nap-5 size-exclusion column with phosphate buffer 25 mM pH 6.2.
Tech 4 - Conjugation at carbohydrates: 25 µL of cargo solution (3.3·10⁻⁹ mol) was diluted with 75 µL of PBS pH 7.2 and mixed with 10µL of 0.1 M NalO₄ in water (54 eq, 1.0·10⁻⁶ mol, 2.1·10⁻⁴ g). The mixture was left to react 2h at 4°C preserved from light. A Nap-5 size exclusion column was used to separate the excess small molecules from the derivatized cargo using phosphate buffer 50 mM pH 6.2. The BBB shuttle peptide bearing an aminooxy moiety (60 eq, 2.0·10⁻⁷ mol) was dissolved in 50 µL of phosphate buffer 25 mM and the pH was made up to 6.2 with 1 M aqueous HCl. The transaminated cargo from the Nap-5 column were mixed with the peptide solution and with 50 µL of a 4·10⁻³ M solution of aniline (60 eq, 2.0·10⁻⁷ mol, 1.9·10⁻⁵ g); the mixture was left to react for 48 h. Excess reagents were removed using a Nap-5 size-exclusion column with phosphate buffer 25 mM pH 6.2.

### Cyclization methods:

Cyclization could be done before or after attaching the cargo to the BBB shuttle.
Cyclization method 1: disulfide bond or Se-Se bond: The cyclization was performed in solution. The peptide was dissolved at a concentration of 100 µM in aqueous ammonium bicarbonate buffer 10 mM and pH 8.0. The solution was intensely stirred for 24 h at room temperature. After that, the product was acidified with TFA to pH 2-3, frozen and lyophilized.
Cyclization method 2: amide bond: The cyclization was performed on resin. The OAII and Alloc groups were first deprotected by addition of tetrakis(triphenylphosphine)palladium(0) (0.1 eq, 10 µM, 12 mg), phenyl silane (10 eq, 1000 µmol, 123 mg) in DCM (3 x 15 min). The resin was washed with 0.02 M sodium diethylcarbamate in DCM (3 x 5 min). The coupling of the amino group of Dap and the carboxylate group of aspartic acid was then achieved by addition of PyBOP (4 eq, 400 µmols, 208 mg), HOAt (12 eq, 1.2 mmol, 163 mg), DMF (1-3 mL/g resin) and DIEA (12 eq, 1.2 mmol, 204 µL). The coupling was left 1.5 h and repeated overnight.
Cyclization method 3: C=C bond: The cyclization is performed on resin. The cyclization is accomplished by olefin metathesis using Grubb's firt generation catalyst as described in G.L. Verdine et al., Methods in Enzymology. 2012. vol. 503. pp. 3-33.
Cyclization method 4: C-C bond: The cyclization is performed on resin. A C=C bond is formed following the cyclization method 3 and the resulting peptide is hydrogenated using Wilkinson's catalyst in dry deoxygenated solvent (10% MeOH/DCM) as described in A.N. Whelan et al., Tetrahedron letters. 2004. vol. 20. pp. 9545-9547.
Cyclization method 5: thioether bond: The thioether bond is accomplished by desulfurization and subsequent cyclization of a cystine bridge as described in J. Malcor et al. J. Med. Chem. 2012. vol 55. pp. 2227-2241.

Characterization of products: The identity of the different peptide compounds synthesized with formula I and II (shuttle or combination of shuttle-peptide cargo) was confirmed using mass spectrometry "Matrix Assisted Laser Desorption/lonization-Mass Spectrometry" (MALDI) (Instrument: MALDI Voyager DE RP time-of-flight (TOF) PE Biosystem) or HPLC-MS (Instrument: Waters model Alliance 2695, quaternary pump, UV/Vis detector model 2998, ESI-MS model Micromass ZQ and software Masslynx version 4.1) using a Sunfire C₁₈ (100 x 2.1 mm x 3.5 µm), 100 Å column using a 0.3 mL/min flow; solvents: A: H₂O with 0.1% formic acid; B: MeCN with 0.07 % formic acid. Their purity was checked by reverse phase HPLC using a Sunfire column (100 x 4.6 mm x 5 µm, 100 Å, Waters) and a Sunfire C₁₈ column (100 x 4.6 mm x 3.5 µm, 100 Å, Waters), flow 1 mL/min; solvents: A: H₂O with 0.045% TFA; B: MeCN with 0.036% TFA, Instrument: Waters model Alliance 2695 constituted by a quaternary pump, a diode array detector model 2998, controlled by the software EmpowerPro 2.

Amino acid analysis: The content and ratio of amino acids present in a peptide-containing sample were determined by ion exchange chromatographic analysis after acid hydrolysis. The hydrolysis was performed with 6M HCl at 110°C during 16h. After that time, the sample was evaporated to dryness at reduced pressure. The residue was dissolved in 20 mM aqueous HCl, derivatized using the AccQ Tag protocol from Waters and finally analyzed by ion exchange HPLC.

Protein conjugates were characterized by HPLC-ESI MS using an LCT-Premier (Waters) with a cromatograph Acquity UPLC binary Sol MGR (Waters) and a column BioSuite pPhenyl 1000RPC 2.0 x 75 mm, 10 µm. Eluents: A: water and B: acetonitrile, both with 0.1 % formic acid. Gradient: 5% to 80% (eluent B) in 60 min and flow rate 100 µL/min.

SDS-PAGE electrophoresis was carried out using BioRad system (Miniprotean cell) 7.5 %Tris gel, 25 mM Tris, 192 mM glycine, 0.1% SDS running buffer). Protein molecular weights were approximated by comparison to a protein marker (Perfect Protein Markers 15-150 kDa from Novagen). Gels were visualised by coomassie staining (staining solution: 10% AcOH, 0.25 g brilliant blue; discoloration solurion: 20%MeOH, 3 % AcOH glacial, in water). Edman sequencing (first 4 cycles) was used to quantify the amount of peptides conjugated at the N-termini.

Peptide compounds were purified by HPLC using a Sunfire C₁₈ column (100 x 19 mm x 5 µm 100 Å, Waters), flow 15 mL/min, solvents: A: H₂O with 0.1% TFA; B: MeCN with 0.1% TFA, Instrument: Waters system with a quaternary pump, Simple Manager 2700 autoinjector, UVNis detector model 2487 and a Fraction collector II, controlled by the software Masslynx version 3.5.

### EXAMPLES

### Example 1: Preparation of H-Cys-Asn-Cys-Lys-Ala-Pro-Glu-Thr-Ala-Leu-Cys-Ala-Ala-Ala-Cys-His-NH₂ (compound of formula (I) where P is SEQ ID NO:9, R₁ is H and Y is NH₂)

For the coupling of the first protected amino acid to the resin, the coupling method 1 was applied using Fmoc-His(Trt)-OH (248 mg).The subsequent amino acids were coupled sequentially as follows using coupling method 1:

| Coupling number | Coupling method | Protected amino acid used | Amount of protected amino acid, mg |
|---|---|---|---|
| 2 | 1 | Fmoc-Cys(Trt)-OH | 234 |
| 3 | 1 | Fmoc-Ala-OH·H₂O | 132 |
| 4 | 1 | Fmoc-Ala-OH·H₂O | 132 |
| 5 | 1 | Fmoc-Ala-OH·H₂O | 132 |
| 6 | 1 | Fmoc-Cys(Trt)-OH | 234 |
| 7 | 1 | Fmoc-Leu-OH | 141 |
| 8 | 1 | Fmoc-Ala-OH·H₂O | 132 |
| 9 | 1 | Fmoc-Thr(Trt)-OH | 136 |
| 10 | 1 | Fmoc-Glu(tBu)-OH | 128 |
| 11 | 1 | Fmoc-Pro-OH | 135 |
| 12 | 2 | Fmoc-Ala-OH·H₂O | 132 |
| 13 | 1 | Fmoc-Lys(Boc)-OH | 182 |
| 14 | 1 | Fmoc-Cys(Trt)-OH | 234 |
| 15 | 1 | Fmoc-Asn(Trt)-OH | 128 |
| 16 | 1 | Fmoc-Cys(Trt)-OH | 234 |

The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.2 min. Mass spectrometry (MALDI-TOF): [M + H]+: 1600.7; Yield (synthesis and purification): 5 %.

### Example 2: Preparation of H-Cys-Lys-Ala-Pro-Glu-Thr-Ala-Leu-Cys-NH₂ (SEQ ID NO:4, R₁ is H and Y is NH₂)

For the coupling of the first protected amino acid to the resin, the coupling method 1 was applied using Fmoc-Cys(Trt)-OH (234 mg). The subsequent amino acids were coupled sequentially as follows using coupling method 1:

| Coupling number | Coupling method | Protected amino acid used | Amount of protected amino acid, mg |
|---|---|---|---|
| 2 | 1 | Fmoc-Leu-OH | 141 |
| 3 | 1 | Fmoc-Ala-OH·H₂O | 132 |
| 4 | 1 | Fmoc-Thr(Trt)-OH | 136 |
| 5 | 1 | Fmoc-Glu(tBu)-OH | 128 |
| 6 | 1 | Fmoc-Pro-OH | 135 |
| 7 | 2 | Fmoc-Ala-OH·H₂O | 132 |
| 8 | 1 | Fmoc-Lys(Boc)-OH | 182 |
| 9 | 1 | Fmoc-Cys(Trt)-OH | 234 |

The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.0 min. Mass spectrometry (MALDI-TOF): [M + H]+: 934.4; Yield (synthesis and purification): 10 %.

### Example 3: Preparation of H-Dap-Lys-Ala-Pro-Glu-Thr-Ala-Leu-Asp-NH₂ (compound of formula (I) where P is SEQ ID NO:12, R₁ is H and Y is NH₂)

For the coupling of the first protected amino acid to the resin, the coupling method 1 was applied using Fmoc-Asp(OAII)-OH (155 mg). The subsequent amino acids were coupled sequentially as follows using coupling method 1:

| Coupling number | Coupling method | Protected amino acid used | Amount of protected amino acid, mg |
|---|---|---|---|
| 2 | 1 | Fmoc-Leu-OH | 141 |
| 3 | 1 | Fmoc-Ala-OH·H₂O | 132 |
| 4 | 1 | Fmoc-Thr(Trt)-OH | 136 |
| 5 | 1 | Fmoc-Glu(tBu)-OH | 128 |
| 6 | 1 | Fmoc-Pro-OH | 135 |
| 7 | 2 | Fmoc-Ala-OH·H₂O | 132 |
| 8 | 1 | Fmoc-Lys(Boc)-OH | 182 |

For the attachement of Fmoc-Dap(Alloc)-OH coupling method 1 was applied using 171 mg of amino acid but the Fmoc group was not removed. The cyclization was performed following cyclization method 2. The N-termial Fmoc group was removed. The peptide was then cleaved and liophilized.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 3.9 min. Mass spectrometry (MALDI-TOF): [M + H]+: 912.5; Yield (synthesis): 40 %.

### Example 4: Preparation of H-Cys-Lys-Ala-Pro-Glu-Thr-Ala-Leu-Cys-NH₂ with a lanthionine bridge linking the two terminal residues of the peptide (compound of formula (I) where P is SEQ ID NO:4, R₁ is H and Y is NH₂ with an intrapeptide thioether bond)

The procedure of Example 2 was repeated until the cyclization step. The cystine (disulfide bond) was then transformed into a lanthionine (thioether bond) by cyclization method 5.

Product characterization. Reverse phase HPLC: linear gradient from 0 to 100% MeCN in H₂O in 8 minutes using an Xselect C₁₈ column (100 mm x 4.6 mm, 3.5 mm, 130 Å, Waters, 20 mm guard column), flow of 1 mL/min; Retention time: 5.8 min. Mass spectrometry (MALDI-TOF): [M + H]+: 900.5; Yield (transformation from Ap5ba after purification): 18 %.

### Example 5: Preparation of H-Lys-Ala-Pro-Glu-Thr-Ala-Leu-NH₂ (compound of formula (I) where P is SEQ ID NO:10, R₁ is H and Y is NH₂)

For the coupling of the first protected amino acid to the resin, the coupling method 1 was applied using Fmoc-Leu-OH (141 mg). The subsequent amino acids were coupled sequentailly as follows:

| Coupling number | Coupling method | Protected amino acid used | Amount of protected amino acid, mg |
|---|---|---|---|
| 2 | 1 | Fmoc-Ala-OH·H₂O | 132 |
| 3 | 1 | Fmoc-Thr(Trt)-OH | 136 |
| 4 | 1 | Fmoc-Glu(tBu)-OH | 128 |
| 5 | 1 | Fmoc-Pro-OH | 135 |
| 6 | 2 | Fmoc-Ala-OH·H₂O | 132 |
| 7 | 1 | Fmoc-Lys(Boc)-OH | 182 |

The peptide was cleaved and liophilized.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 3.8 min. Mass spectrometry (MALDI-TOF): [M + H]+: 728.5; Yield (synthesis and purification): 30 %.

### Example 6: Preparation of H-C≡C(CH₂)ᵣC(C=O)-Cys-Asn-Cys-Lys-Ala-Pro-Glu-Thr-Ala-Leu-Cys-Ala-Ala-Ala-Cys-His-NH₂ (compound of formula (I) where P is SEQ ID NO:9, V is -C≡C(CH₂)ᵣC(C=O)-, R₁ is H, and Y is NH₂)

The procedure of the Example 1 was repeated until the coupling of the sixteenth amino acid. For the coupling of the hexynoic acid to the BBB-shuttle anchored onto the resin the following protocol was used: hexynoic acid (4 eq, 400 µmols, 45 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1 h. The solvent was removed by suction, the resin washed with DMF (5 x 30 s) and DCM (5 x 30 s). The coupling was repeated under the same conditions. The extent of coupling was monitored using the Kaiser colorimetric assay. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.6 min. Mass spectrometry (MALDI-TOF): [M + H]+: 1695.0; Yield (synthesis and purification): 4 %.

### Example 7: Preparation of H-C≡C(CH₂)ᵣC(C=O)-Cys-Lys-Ala-Pro-Glu-Thr-Ala-Leu-Cys-NH₂ (compound of formula (I) where P is SEQ ID NO:4, V is-C≡C(CH₂)ᵣC(C=O)-, R₁ is H, and Y is NH₂)

The procedure of the Example 2 was repeated until the coupling of the ninth amino acid. For the coupling of the hexynoic acid to the BBB-shuttle anchored onto the resin the following protocol was used: hexynoic acid (4 eq, 400 µmols, 45 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.5 min. Mass spectrometry (MALDI-TOF): [M + H]+: 1048.6; Yield (synthesis): 35 %.

### Example 8: Preparation of maleimide-(CH₂)₆-C(=O)-Dap-Lys-Ala-Pro-Glu-Thr-Ala-Leu-Asp-NH₂ (compound of formula (I) where P is SEQ ID NO:12, V is-(CH₂)₆-C(=O)-, R₁ is maleimide, and Y is NH₂)

The procedure of Example 2 was repeated until the cyclization step. For the coupling of the maleimidohexanoic acid to the BBB-shuttle anchored onto the resin the following protocol was used: he Fmoc protecting group was removed, then we added maleimidohexanoic acid (4 eq, 400 µmols, 45 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The extent of coupling was monitored using the Kaiser colorimetric assay. The peptide was cleaved and liophilized.

Product characterization. Reverse phase HPLC: linear gradient from 0 to 100% MeCN in H₂O in 8 minutes using an Sunfire C₁₈ column (100 mm x 4.6 mm, 3.5 mm, 100 Å, Waters), flow of 1 mL/min; Retention time: 3.7 min. Mass spectrometry (MALDI-TOF): [M + H]+: 1105.2; Yield (after synthesis and purification): 10 %.

### Example 9: Preparation of H-Cys-Dap-Lys-Ala-Pro-Glu-Thr-Ala-Leu-Asp-NH₂ (compound of formula (I) where P is SEQ ID NO:12, V is S-CH₂-CH(NH₂)-C(=O),, R₁ is H, and Y is NH₂)

The procedure of Example 3 was repeated until the cyclization step. For the coupling of the Fmoc-Cys(Trt)-OH to the BBB-shuttle anchored onto the resin the following protocol was used: the Fmoc protecting group from the peptide was removed, then Fmoc-Cys(Trt)-OH was added (4 eq, 400 µmols, 234 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The extent of coupling was monitored using the Kaiser colorimetric assay. The Fmoc group of Cys was removed. The peptide was cleaved and liophilized.

Product characterization. Reverse phase HPLC: linear gradient from 0 to 100% MeCN in H₂O in 8 minutes using an Sunfire C₁₈ column (100 mm x 4.6 mm, 3.5 mm, 100 Å, Waters), flow of 1 mL/min; Retention time: 3.7 min. Mass spectrometry (MALDI-TOF): [M + H]+: 1015.5; Yield (after synthesis and purification): 9 %.

### Example 10: Preparation of 5(6)-Carboxyfluorescein-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9 and 5(6)-Carboxifluorescein as cargo or Z group)

The procedure of the Example 1 was repeated until the coupling of the sixteenth amino acid. For the coupling of the (5)6-carboxyfluorescein to the BBB-shuttle anchored onto the resin the following protocol was used: 5(6)-carboxyfluorescein (4 eq, 400 µmols, 151 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 |uL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The benzyl esters that might have formed were removed by three treatments of 1 min with 20% solution of piperidine in DMF (v/v). The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 5.0 min. Mass spectrometry (MALDI-TOF): [M + H]+: 1962.9; Yield (synthesis and purification): 3 %.

### Example 11: Preparation of 5(6)-Carboxyfluorescein-Gly-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9, Gly as V and 5(6)-Carboxifluorescein as cargo or Z group)

The procedure of the Example 1 was repeated until the coupling of the sixteenth amino acid. Fmoc-Gly-OH was then linked using coupling method 1. For the coupling of the (5)6-carboxyfluorescein to the BBB-shuttle anchored onto the resin the following protocol was used: 5(6)-carboxyfluorescein (4 eq, 400 µmols, 151 mg) in DMF (1-3 mL/g resin), PyBOP (4 eq, 400 µmols, 208 mg) and HOAt (12 eq, 1.2 mmol, 204 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The benzyl esters that might have formed were removed by three treatments of 1 min with 20% solution of piperidine in DMF (v/v). The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 5.0 min. Mass spectrometry (MALDI-TOF): [M + H]+: 2019.8; Yield (synthesis and purification): 5 %.

### Example 12: Preparation of 5(6)-Carboxyfluorescein-Gly-Dap-Lys-Ala-Pro-Glu-Thr-Ala-Leu-Cys-Asp-NH₂ (Compound of formula (II) comprising SEQ ID NO:12, Gly as V and 5(6)-Carboxifluorescein as cargo or Z group)

The procedure of the Example 3 was repeated until the coupling of the ninth amino acid. After removing the Fmoc group, a glycine was then linked using coupling method 1. For the coupling of the (5)6-carboxyfluorescein to the BBB-shuttle anchored onto the resin the following protocol was used: 5(6)-carboxyfluorescein (4 eq, 400 µmols, 151 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The benzyl esters that might have formed were removed by three treatments of 1 min with 20% solution of piperidine in DMF (v/v). The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 5.0 min. Mass spectrometry (MALDI-TOF): [M + H]+: 1327.6; Yield (synthesis and purification): 7 %.

### Example 13 Preparation of sulforhodamine b-Gly-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9, Gly as V and sulforhodamine b as cargo or Z group)

The procedure of the Example 1 was repeated until the coupling of the sixteenth amino acid. For the coupling of the spacer (Gly) to the BBB-shuttle anchored onto the resin the coupling method 1 was applied using Fmoc-Gly-OH (4 eq. 400 µmols, 119 mg), TBTU (4 eq, 400 µmols, 128 mg).

For the coupling of the sulforhodamine b to the H-Gly-BBB-shuttle anchored onto the resin the following protocol was used: sulforhodamine b (2 eq, 200 µmols, 115 mg) in DMF (1-3 mL/g resin) was added to the resin followed by the addition of 4 eq of DIEA (400 µmol, 68 µL). The mixture was allowed to react overnight at 50 °C. The solvent was removed by suction, the resin washed with DMF (5 x 30 s) and DCM (5 x 30 s). The coupling was repeated under the same conditions. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 5.3 min. Mass spectrometry (MALDI-TOF): [M + H]+: 2199.0; Yield (synthesis and purification): 1 %.

### Example 14: Preparation of sulforhodamine b-Gly-Cys-Lys-Ala-Pro-Glu-Thr-Ala-Leu-Cys-NH₂ (Compound of formula (II) comprising SEQ ID NO:4, Gly as V and sulforhodamine b as cargo or Z group)

The procedure of the Example 2 was repeated until the coupling of the ninth amino acid. For the coupling of the spacer (Gly) to the BBB-shuttle anchored onto the resin the coupling method 1 was applied using Fmoc-Gly-OH (4 eq. 400 µmols, 119 mg), TBTU (4 eq, 400 µmols, 128 mg).

For the coupling of the sulforhodamine b to the H-Gly-BBB-shuttle anchored onto the resin the following protocol was used: sulforhodamine b (2 eq, 200 µmols, 115 mg) in DMF (1-3 mL/g resin) was added to the resin followed by the addition of 4 eq of DIEA (400 µmol, 68 µL). The mixture was allowed to react overnight at 50 °C. The solvent was removed by suction, the resin washed with DMF (5 x 30 s) and DCM (5 x 30 s). The coupling was repeated under the same conditions. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 5.3 min. Mass spectrometry (MALDI-TOF): [M + H]+: 1531.6; Yield (synthesis): 4 %.

### Example 15: Preparation of biotin-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9, and biotin as cargo or Z group)

The procedure of the Example 1 was repeated until the coupling of the sixteenth amino acid. For the coupling of the biotin to the BBB-shuttle anchored onto the resin the following protocol was used: biotin (4 eq, 400 µmols, 98 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 A, Waters), flow of 1 mL/min; Retention time: 4.6 min. Mass spectrometry (MALDI-TOF): [M + H]+: 1868.9; Yield (synthesis and purification): 5 %.

### Example 16: Preparation of L-Dopa-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9 and L-Dopa as cargo or Z group)

The procedure of the Example 1 was repeated until the coupling of the sixteenth amino acid. For the coupling of the L-dopa to the BBB-shuttle anchored onto the resin the following protocol was used: L-dopa (4 eq, 400 µmols, 79 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.2 min. Mass spectrometry (MALDI-TOF): [M + H]+: 1779.8; Yield (synthesis and purification): 4 %.

### Example 17: Preparation of L-Dopa-Cys-Lys-Ala-Pro-Glu-Thr-Ala-Leu-Cys-NH₂ (Compound of formula (II) comprising SEQ ID NO:4 and L-Dopa as cargo or Z group)

The procedure of the Example 2 was repeated until the coupling of the ninth amino acid. For the coupling of the L-dopa to the BBB-shuttle anchored onto the resin the following protocol was used: L-dopa (4 eq, 400 µmols, 79 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.0 min. Mass spectrometry (MALDI-TOF): [M + H]+: 1111.6; Yield (synthesis): 20 %.

### Example 18: Preparation of L-Dopa-Dap-Lys-Ala-Pro-Glu-Thr-Ala-Leu-Asp-NH₂ (Compound of formula (II) comprising SEQ ID NO: 12 and L-Dopa as cargo or Z group)

The procedure of the Example 3 was repeated until the coupling of the ninth amino acid and the cyclization. For the coupling of the L-dopa to the BBB-shuttle anchored onto the resin the following protocol was used: Fmoc group was deprotected, L-dopa (4 eq, 400 µmols, 79 mg) in DMF (1-3 mL/g resin), PyBOP (4 eq, 400 µmols, 208 mg) and HOAt (12 eq, 1.2 mmol, 163 mg) were sequentially added to the resin followed by the addition of 12 eq of DIEA (1.2 mmol, 204 µL). The mixture was allowed to react with intermittent manual stirring for 1.5 h. The solvent was removed by suction, the resin washed with DMF (5 x 30 s) and DCM (5 x 30 s). The coupling was repeated under the same conditions. The extent of coupling was monitored using the Kaiser colorimetric assay. The peptide was cleaved and liophilized. The cyclization was performed following cyclization method 1.

Product characterization. Reverse phase HPLC: linear gradient from 0 to 100% MeCN in H₂O in 8 minutes using a Xselect C₁₈ column (100 mm x 4.6 mm, 3.5 mm, 130 Å, Waters), flow of 1 mL/min; Retention time: 5.2 min. Mass spectrometry (MALDI-TOF): [M + H]+: 1091.6; Yield (synthesis and purification): 8 %.

### Example 19: Preparation of H-Leu-Pro-Phe-Phe-Asp-Gly-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:13 which corresponds to SEQ ID NO:9, Gly as V and Leu-Pro-Phe-Phe-Asp (SEQ ID NO: 11) as cargo or Z group)

The procedure of the Example 1 was repeated until the coupling of the ninth amino acid. For the coupling of the spacer (Gly) (coupling 17) to the BBB-shuttle anchored onto the resin the coupling method 1 was applied using Fmoc-Gly-OH (4 eq. 400 µmols, 119 mg), TBTU (4 eq, 400 µmols, 128 mg).

The amino acids of the cargo were coupled sequentially as follows:

| Coupling number | Coupling method | Protected amino acid used | Amount of protected amino acid, mg |
|---|---|---|---|
| 18 | 1 | Fmoc-Asp(tBu)-OH | 165 |
| 19 | 1 | Fmoc-Phe-OH | 212 |
| 20 | 2 | Fmoc-Phe-OH | 212 |
| 21 | 1 | Fmoc-Pro-OH | 135 |
| 22 | 2 | Fmoc-Leu-OH | 141 |

The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.8 min. Mass spectrometry (MALDI-TOF): [M + H]+: 2276.9; Yield (synthesis and purification): 3 %.

### Example 20: Preparation of H-Leu-Pro-Phe-Phe-Asp-Gly-Cys-Lys-Ala-Pro-Glu-Thr-Ala-Leu-Cys-NH₂ (Compound of formula (II) comprising SEQ ID NO: 15 which corresponds to SEQ ID NO:4. Gly as V and Leu-Pro-Phe-Phe-Asp (SEQ ID NO:11) as cargo or Z group)

The procedure of the Example 2 was repeated until the coupling of the sixteenth amino acid. For the coupling of the spacer (Gly) (coupling 10) to the BBB-shuttle anchored onto the resin the coupling method 1 was applied using Fmoc-Gly-OH (4 eq. 400 µmols, 119 mg), TBTU (4 eq, 400 µmols, 128 mg).

The amino acids of the cargo were coupled sequentially as follows:

| Coupling number | Coupling method | Protected amino acid used | Amount of protected amino acid, mg |
|---|---|---|---|
| 11 | 1 | Fmoc-Asp(tBu)-OH | 165 |
| 12 | 1 | Fmoc-Phe-OH | 212 |
| 13 | 2 | Fmoc-Phe-OH | 212 |
| 14 | 1 | Fmoc-Pro-OH | 135 |
| 15 | 2 | Fmoc-Leu-OH | 141 |

The peptide was cleaved and liophilized. The cyclization was performed following cyclization method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 4.9 min. Mass spectrometry (MALDI-TOF): [M + H]+: 1610.9; Yield (synthesis): 30 %.

### Example 21: Preparation of Lysozyme-V₃-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9, V₃ as V and lysozyme as cargo or Z group)

Example 6 was repeated until the cleavage step. The coupling of lysozyme through lysine residues was performed in solution using the conjguation by an alkyne-azide cycloaddition (Tech 1). The MALDI-TOF MS spectrum revealed the formation of conjugates bearing 0-5 peptides with an average of 0.4 peptides/lysozyme molecule.

### Example 22: Preparation of Cetuximab-V₃-Cys-Asn-Cys-Lys-Ala-Pro-Glu-Thr-Ala-Leu-Cys-Ala-Ala-Ala-Cys-His-NH₂ (Compound of formula (II) comprising SEQ ID NO:9, V₃ as V and Cetuximab as cargo or Z group)

Example 6 was repeated until the cleavage step. The coupling of cetuximab through lysine residues was performed in solution using the conjguation by an alkyne-azide cycloaddition (Tech 1). The HPLC-ESI mass spectrum and SDS-PAGE revealed the formation of conjugates bearing 0-6 peptides with an average of 2 peptides/cetuximab molecule.

### Example 23: Preparation of Cetuximab-V₂-Dap-Lys-Ala-Pro-Glu-Thr-Ala-Leu-Asp-NH₂ (Compound of formula (II) comprising SEQ ID NO:12, V₂ as V and Cetuximab as cargo or Z group)

Example 8 was repeated until the cleavage step. The coupling of cetuximab through the partially reduced interchain cysteinee residues was performed in solution using Tech 2.1. The HPLC-ESI mass spectrum and SDS-PAGE revealed the formation of conjugates bearing 0-4 peptides with an average of 2 peptides/cetuximab molecule.

### Example 24: Preparation of Quantum dot-PEG-NH-V₁-Dap-Lys-Ala-Pro-Glu-Thr-Ala-Leu-Asp-NH₂ (Compound of formula (II) comprising SEQ ID NO:12, V₁ as V and Quantum dot-PEG-NH₂ as cargo or Z group)

Example 9 was repeated until the cleavage step. The coupling of Quantum dot-PEG-NH₂ was performd as described by W. Cai et al., Nature Protocols 2008, vol.3, p89-96. 52 peptides per quantum dot were quantified using amino acid analysis.

### Example 25: Preparation of Gold nanoparticle-Cys-Dap-Lys-Ala-Pro-Glu-Thr-Ala-Leu-Asp-NH₂ (Compound of formula (II) comprising SEQ ID NO:12, Cys as V and Cetuximab as cargo or Z group)

Example 9 was repeated until the cleavage step. Gold nanoparticle synthesis and conjugation to the peptide shuttle was performed as described by Prades et al. Biomaterials 2012, vol33, pp. 7194-7205. 1205 peptides per gold nanoparticle were quantified using amino acid analysis.

### Example 26: Preparation of H-Cys-Asn-Cys-Lys-Ala-Pro-Glu-Thr-Ala-Leu-Cys-Ala-Ala-Ala-Cys-His-Gly-Leu-Pro-Phe-Phe-Asp-NH₂ (Compound of formula (II) comprising SEQ ID NO: 14 which corresponds to SEQ ID NO:9, Gly as V and Leu-Pro-Phe-Phe-Asp-NH₂ as cargo at the C-terminus or Z group)

For the coupling of the first protected amino acid to the resin, the coupling method 1 was applied using Fmoc-His(Trt)-OH (248 mg). The subsequent amino acids were coupled sequentially as follows using coupling method 1:

| Coupling number | Coupling method | Protected amino acid used | Amount of protected amino acid, mg |
|---|---|---|---|
| 2 | 1 | Fmoc-Cys(Trt)-OH | 234 |
| 3 | 1 | Fmoc-Ala-OH·H₂O | 132 |
| 4 | 1 | Fmoc-Ala-OH·H₂O | 132 |
| 5 | 1 | Fmoc-Ala-OH·H₂O | 132 |
| 6 | 1 | Fmoc-Cys(Trt)-OH | 234 |
| 7 | 1 | Fmoc-Leu-OH | 141 |
| 8 | 1 | Fmoc-Ala-OH·H₂O | 132 |
| 9 | 1 | Fmoc-Thr(Trt)-OH | 136 |
| 10 | 1 | Fmoc-Glu(tBu)-OH | 128 |
| 11 | 1 | Fmoc-Pro-OH | 135 |
| 12 | 2 | Fmoc-Ala-OH·H₂O | 132 |
| 13 | 1 | Fmoc-Lys(Boc)-OH | 182 |
| 14 | 1 | Fmoc-Cys(Trt)-OH | 234 |
| 15 | 1 | Fmoc-Asn(Trt)-OH | 128 |
| 16 | 1 | Fmoc-Cys(Trt)-OH | 234 |
| 17 | 1 | Fmoc-Gly(Trt)-OH | 119 |
| 18 | 1 | Fmoc-Asp(tBu)-OH | 165 |
| 19 | 1 | Fmoc-Phe-OH | 212 |
| 20 | 2 | Fmoc-Phe-OH | 212 |
| 21 | 1 | Fmoc-Pro-OH | 135 |
| 22 | 2 | Fmoc-Leu-OH | 141 |

The peptide was cleaved and liophilized. The cyclization was performed following cyclization method 1.

The cleavage step was performed treating the resin with a mixture of TFA (95%) and H2O (2.5%) (1 x 2 h). tert-butyl methyl ether was added to the product obtained and the mixture was centrifuged (3 x 8 min). The supernatant was removed and the pellet resuspended in a mixture of H2O, MeCN and TFA (1000:1000:1). The product was filtered, frozen and lyophilized. The cyclization was performed following method 1.

Product characterization. Reverse phase HPLC: linear gradient from 0 to 100% MeCN in H₂O in 8 minutes using a Sunfire C₁₈ column (100 mm x 4.6 mm, 3.5 mm, 100 Å, Waters), flow of 1 mL/min; Retention time: 5.0 min. Mass spectrometry (MALDI-TOF): [M + H]+: 2276.9; Yield (synthesis and purification): 7 %.

### Example 27: Preparation of H-Dap-Lys-Ala-Pro-Glu-Thr-Ala-Leu-Asp-Lys-Gln-Ile-Glu-Ile-Lys-Lys-Phe-Lys-NH₂ (Compound of formula (II) comprising SEQ ID NO:19 which corresponds to SEQ ID NO:12 and Lys-Gln-Ile-Glu-lle-Lys-Lys-Phe-Lys-NH₂ (SEQ ID NO:20) as cargo at the C-terminus or Z group)

For the coupling of the first protected amino acid to the resin, the coupling method 1 was applied using Fmoc-Lys(Boc)-OH (182 mg).

The subsequent amino acids were coupled sequentially as follows using coupling method 1:

| Coupling number | Coupling method | Protected amino acid used | Amount of protected amino acid, mg |
|---|---|---|---|
| 2 | 1 | Fmoc-Phe-OH | |
| 3 | 1 | Fmoc-Lys(Boc)-OH | 182 |
| 4 | 1 | Fmoc-Lys(Boc)-OH | 182 |
| 5 | 1 | Fmoc-Ile-OH | 141 |
| 6 | 1 | Fmoc-Glu(tBu)-OH | 170 |
| 7 | 1 | Fmoc-Ile-OH | 141 |
| 8 | 1 | Fmoc-Gln(Trt)-OH | 244 |
| 9 | 1 | Fmoc-Lys(Boc)-OH | 128 |
| 10 | 1 | Fmoc-Asp(Alloc)-OH | 165 |
| 11 | 1 | Fmoc-Leu-OH | 141 |
| 12 | 1 | Fmoc-Ala-OH·H₂O | 132 |
| 13 | 1 | Fmoc-Thr(Trt)-OH | 136 |
| 14 | 1 | Fmoc-Glu(tBu)-OH | 128 |
| 15 | 1 | Fmoc-Pro-OH | 135 |
| 16 | 2 | Fmoc-Ala-OH·H₂O | 132 |
| 17 | 1 | Fmoc-Lys(Boc)-OH | 182 |

The Fmoc-Dap(Alloc)-OH was coupled using 164 mg of protected amino acid using coupling method 1 but without removing Fmoc. The cyclization was performed following cyclization method 2. The peptide was cleaved and liophilized.

The cleavage step was performed treating the resin with a mixture of TFA (95%) and H2O (2.5%) (1 x 2 h). Tert-butyl methyl ether was added to the product obtained and the mixture was centrifuged (3 x 8 min). The supernatant was removed and the pellet resuspended in a mixture of H2O, MeCN and TFA (1000:1000:1). The product was filtered, frozen and lyophilized. The cyclization was performed following method 1.

Product characterization. Reverse phase HPLC: linear gradient from 0 to 100% MeCN in H₂O in 8 minutes using a Xselect C₁₈ column (100 mm x 4.6 mm, 3.5 mm, 130 Å, Waters, 20 mm guard columng), flow of 1 mL/min; Retention time: 5.9 min. Mass spectrometry (MALDI-TOF): [M + H]+: 2055.2; Yield (synthesis and purification): 6 %.

### Example 28: Evaluation of transport across the BBB using an in vitro bovine cell assay

The evaluation method chosen to test the compounds of formula (I) and (II) of the invention was a cell-based assay consisting of a co-culture of brain endothelial cells and astrocytes, which allows to evaluate or to predict the transport of compounds through the BBB. The co-culture method used was described by Gaillard and de Boer in 2008 (Gaillard, PJ et al. "2B-Trans technology: targeted drug delivery across the blood-brain barrier" Methods Mol Biol 2008, vol. 437 pp. 161-175) and consists in seeding of rat astrocytes in a side of a polycarbonate filter (transwell) and bovine brain endothelial cells on the other side of the filter. Under these conditions endothelial cells are able to reach the confluence mimicking the BBB. The system allows studying the transport of compounds that can cross the BBB by a passive transport mechanism and/or active. Is commonly used model for permeation studies and toxicity of compounds across the BBB.

The co-culture assay was used to determine the capacity of the constructs cargo-shuttle to cross the BBB. The apparent permeability of the compounds was measured in triplicate at a concentration of 200 µ M for Examples 1, 2, 3, 5, 10, 13, 15, 16, 17 and 19 and at a concentration of 1.75 µM for Example 21. All the assays were performed with transendothelial electrical resistance values equal or higher than 100 Ω · cm² and were carried out in the presence of lucifer yellow to assess the integrity of the cell membrane during the test. The permeability tests were performed in Ringer-HEPES buffer at pH 7.4. The compound of interest was dissolved in the buffer to the desired concentration.

Once the endothelial cells were confluent on the filters (transwells) these were washed with Ringer-HEPES buffer. The acceptor well was filled with 800 µ L of Ringer-HEPES buffer and the donor well with 200 µL of the solution of the compound to be studied. The system was incubated at 37 °C and 5% CO2 for 2 h. After that time the content of the acceptor and donor compartments were analyzed by HPLC-UV, direct ELISA or fluorescence (depending on cargo anchored to the shuttle). Transport was also confirmed by mass spectrometry MALDI-TOF for all peptides. The apparent permeability was calculated using the following equation: Papp = (dQ/dt) * (1/A) * (1/CO) (cm/s), where (dQ/dt) is the amount of compound present in the compartment acceptor function of time (nmol/s). A is the area of the filter(cm2) and CO is the initial concentration of the compound applied in the donor compartment (nmol/mL). Table 2 shows that all BBB-shuttle-cargo constructs cross the blood-brain barrier effectively (Papp > 10⁻⁶ cm/s).

Table 2: Apparent permeability (Papp) and percentage of transport after a 2 hours assay in the bovine cell model for Examples 1, 2, 3, 5, 10, 13, 15, 16, 17, 19 and 21. The cargos L-dopa, biotin, 5(6)-carboxyfluorescein, sulforhodamine b, the peptide H-Leu-Pro-Phe-Phe-Asp-NH₂ and lysozyme were not detected in the acceptor compartments by MALDI-TOF MS after the 2 h assay.
Comparative example 1 clearly shows that apamin has a significantly lower permeability (p < 0.0001, unpaired t-test) than the peptide shuttles here described (examples 1, 2, 3, 4 and 5).
Comparative example 2 shows that the peptide shuttles here described enhance the transport of small proteins such as lysozyme in spite of a low conjugation yield (example 12).

| Reference Compound | Compound | Papp (x 10⁶) cm/s | Transport (%) (2h) |
|---|---|---|---|
| Comparative example 1 (SEQ ID NO: 16) | Apamin: H-Cys-Asn-Cys-Lys-Ala-Pro-Glu-Thr- Ala-Leu-Cys-Ala-Arg-Arg-Cys-Gln-Gln-His-NH₂ | 0.8±0.1 | 0.9±0.2 |
| Comparative example 2: | Lysozyme | 0.05±0.01 | 0.06±0.02 |
| Example 1: | H-Ap3-NH₂: H-Cys-Asn-Cys-Lys-Ala-Pro-Glu-Thr-Ala-Leu-Cys-Ala-Ala-Ala-Cys-His-NH₂ (Cys1-Cys1 and Cys3-Cys11, disulfide bonds) | 2.7±0.3 | 3.2±0.3 |
| Example 2: | H-Ap5c-NH₂: H-Cys-Lys-Ala-Pro-Glu-Thr-Ala-Leu-Cys-NH₂ (Cys-Cys, disulfide bond) | 2.6±0.8 | 3.1±1.0 |
| Example 3: | H-Ap5a-NH₂: H-Dap- -Lys-Ala-Pro-Glu-Thr-Ala-Leu-Asp-NH₂ (Dap-Asp, amide bond) | 5.1±0.6 | 6.0±0.7 |
| Example 5: | H-Lys-Ala-Pro-Glu-Thr-Ala-Leu-NH₂ | 2.0±0.6 | 2.3±0.8 |
| Example 10: | (5)6-carboxyfluorescein-Ap3-NH₂ | 3.1±1.5 | 3.7±1.8 |
| Example 13: | Sulforhodamine b-Gly-Ap3-NH₂ | 2.0±0.3 | 2.4±0.4 |
| Example 15: | biotin-Ap3-NH₂ | 1.6±0.6 | 1.9±0.8 |
| Example 16: | L-dopa-Ap3-NH₂ | 2.6±0.3 | 3.1±0.3 |
| Example 17: | L-dopa-Ap5-NH₂ | 2.4 | 2.9 |
| Example 19: | H-Leu-Pro-Phe-Phe-Asp-Gly-Ap3-NH₂ | 1.5±0.4 | 1.8±0.5 |
| Example 21: | Lysozyme-(CH=C)-(CH2)3-C(=O)-Ap3-NH₂ | 0.07±0.03 | 0.08±0.03 |

### Example 29: Evaluation of transport across the BBB using an in vitro human cell assay

Due to the great interest to know whether the cargo-BBB shuttle conjugates would be transported across human brain endothelial cells, some of the most relevant cargoes for therapy and diagnosis were assayed in a novel human-based BBB cell model. In this case, the co-culture method used was described by Cecchelli et al. (Cecchelli et al. PloS One. 2014. vol9. pp. e99733) and consists in seeding bovine pericytes in the bottom of a 12-well plate and human endothelial cells derived from hematopoietic stem cells on a transwell filter. Under these conditions endothelial cells are able to reach the confluence mimicking the human BBB. The system allows studying the transport of compounds that can cross the BBB by a passive transport mechanism and/or active.

The apparent permeability of the compounds was measured in triplicate at a concentration of 200 µM for Examples 3, 4, 5 and 27, 25 nM Examples 22 and 23, 30 nM for Example 24 and 5 nM for example 25. All the assays were carried out in the presence of lucifer yellow to assess the integrity of the cell membrane during the test. The permeability tests were performed in Ringer-HEPES buffer at pH 7.4. The compound of interest was disolved or buffer-exhanged and diluted to the desired concentration.

Cetuximab and cetuximab conjugates were radiolabelled to be assayed in the cell model. Pierce^{(R)} iodination beads were used following the instructions provided by the manufacturer.

Once the endothelial cells were confluent on the filters (transwells) these were washed with Ringer-HEPES buffer. The acceptor well was filled with 1500 µ L of Ringer-HEPES buffer and the donor well with 500 µL of the solution of the compound to be studied. The system was incubated at 37 °C and 5% CO2 for 2 h. After that time the content of the acceptor and donor compartments were analyzed by HPLC-UV, gamma counting or ICP-MS (depending on cargo anchored to the shuttle). Transport was also confirmed by mass spectrometry MALDI-TOF for all peptides. The apparent permeability was calculated using the same equation as for the bovine cell model.

Table 3: Apparent permeability (Papp) and percentage of transport after a 2 hours assay in the human cell model for Examples 2, 4, 18, 22, 23, 24, 25 and 27.

Example 3 assayed in the human model shows that permeability is comparable. However, values in the human model are considered more relevant because the receptors and active mechanisms are more similar to those found in human beings.
Comparative example 3 shows that the peptide shuttles here described enhance the transport of large therapeutic proteins such as monoclonal antibodies (examples 13 and 18)
Comparative example 4 shows that the peptide shuttles here described enhance the transport of nanoparticles with great potential for diagnosis such as quantum dots (example 19)
Comparative example 5 shows that the peptide shuttles here described enhance the transport of nanoparticles with great potential for therapeutics such as gold nanoparticles (example 20)

| Reference Compound | Compound | Papp (x 10⁶) cm/s | Transport (%) (2h) |
|---|---|---|---|
| Comparative example 3: | Cetuximab | 1.3±0.1 | 2.3±0.2 |
| Comparative example 4: | QD-PEG-NH₂ | 0.08±0.03 | 0.14±0.05 |
| Comparative example 5: | Au_nanoparticles | Not detected | Not detected |
| Example 3: | H-Ap5a-NH₂: H-Dap-Lys-Ala-Pro-Glu-Thr-Ala-Leu-Asp-NH₂ (Dap-Asp, amide bond) | 6.7±0.6 | 10.9±1.1 |
| Example 4: | H-Ap5l-NH₂: H-Cys-Lys-Ala-Pro-Glu-Thr-Ala-Leu-Cys-NH₂ (Cys-Cys, lanthionine)* | 6.1±0.4 | 9.9±0.6 |
| Example 18: | L-dopa-Ap5a-NH₂ | 4.0±1.3 | 6.6±2.1 |
| Example 22: | Cetuximab-V₃-Ap3-NH₂ | 9.2±0.5 | 15.9±0.9 |
| Example 23: | Cetuximab-V₂-Ap5a-NH₂ | 2.4±0.2 | 4.1±0.4 |
| Example 24: | QD-PEG-NH-V₁-Ap5a-NH₂ | 0.13±0.05 | 0.23±0.09 |
| Example 25: | Au_nanoparticle-Cys-Ap5a-NH₂ | 0.020 | 0.034 |
| | | ±0.002 | ±0.004 |
| Example 27: | H-Ap5a-Lys-Gln-Ile-Glu-Ile-Lys-Lys-Phe-Lys-NH₂ | 1.9±0.1 | 3.1±0.2 |

| | | | |
|---|---|---|---|
| V₁, V₂ and V₃ are linkers as defined above. The values of permeability equal to or higher than 1·10⁻⁶ cm/s are considered exceptionally good, and values equal to or higher than 0.1·10⁻⁶ cm/s are considered good. The permeability values depend highly on the cargo used. For a large cargo such as lysozyme, antibodies, quantum dots and gold nanoparticles, what is important is the increase in transport with respect to the cargo without BBB shuttle. Transporting some of this cargoes has proved to be extremely challenging. Therefore, any increase is remarkable as small as it may seem. | | | |

### Experiment 30: Evidences of active transport

1) NaN₃ inhibition: Sodium azide (NaN₃) is an inhibitor of the resipiratory pathway and, therefore, decreases energy-dependent processes such as transcytosis. The transport of SEQ ID NO9 was very significantly (p < 0.001) inhibited by a 30 min treatment with 1.5 mM sodium azide prior to the permeability assay.
2) saturation: Three concentrations of the compound with SEQ ID NO9 were assayed following the procedure of the Example 28.

| Concentration /µM | Relative transport |
|---|---|
| 100 | 100±39 |
| 200 | 110±6 |
| 500 | 86±6 |

The results are consistent with a saturable transport mechanism considering the plateau region has been reached at 100 µM.
3) dramatic decrease in permeability at reduced temperature: The procedure of the Example 28 was followed until the endothelial cells were confluent. To perform the experiment, the filters (transwells) were washed with Ringer-HEPES buffer. The abluminal compartment was filled with 800 µL of Ringer-HEPES and the luminal compartment was filled with 200 µL of the compound with SEQ ID NO 9 in Ringer-HEPES (200 µM). The system was incubated at 4 °C and 5 % CO2 for 2 h. After that time the content of the acceptor and donor compartments were analyzed by HPLC-UV. Transport was also confirmed by mass spectrometry MALDI-TOF for all peptides. The permeability at 4 °C is 0.23±0.04)·10⁻⁶ cm/s, which is dramatically lower (P < 0.0001, unpaired t-test) than at 37 °C. This low transport is consistent with an energy-dependent process.
4) luminal-abluminal transport > abluminal-luminal transport.: The procedure of the Example 28 was followed until the endothelial cells were confluent. To perform the experiment, the filters (transwells) these were washed with Ringer-HEPES buffer. The abluminal compartment (in this experiment the donor) was filled with 800 µL of the compound with SEQ ID NO 9 in Ringer-HEPES (200 µM) and the luminal compartment (in this experiment the acceptor) with 200 µL Ringer-HEPES. The system was incubated at 37 °C and 5 % CO2 for 2 h. After that time the content of the acceptor and donor compartments were analyzed by HPLC-UV. Transport was also confirmed by mass spectrometry MALDI-TOF for all peptides.

The abluminal to luminal transport is (1.06±0.03)·10⁻⁶ cm/s, which is significantly lower (P < 0.001, unpaired t-test) than luminal to abluminal transport. This asymmetry in the transport indicates that it is not due to passive diffusion and that the compound is not a good substrate for an efflux pump.

### Example 31: Evidence of active transport: the PAMPA assay was used to determine the inability of compound with SEQ ID NO 9 to cross the BBB by passive diffusion.

The buffer was prepared from the concentrated solution by plON, following the manufacturer's instructions. The pH was adjusted to 7.4 using a solution of 0.5 M NaOH. The PAMPA sandwich was separated, and the donor well was filled with 195 µL of the compound with SEQ ID NO 9 (400 µM) solution and a stirring magnet was added. The acceptor plate was then placed into the donor plate. Next, 4 µL of the 20 mg/mL phospholipid mixture (PBLEP) in dodecane was added to the filter of each well, and 200 µL of buffer solution was added to each acceptor well. The plate was then covered. Four replicates were performed for each compound. The PAMPA plate was incubated for 4 h at 20°C and under saturated atmosphere in a GUT-BOXTM apparatus under an agitation vigorous enough to reduce the unstirred water layer (UWL) to 25 µm. After the incubation, the sandwich was separated, and an aliquot of each donor and acceptor well was analyzed by RP-HPLC-UV ad MALDI-TOF MS.

The compound could not be detected by HPLC-UV and only in two out of four replicates was found by MALDI-TOF MS. As the PAMPA is a model for passive diffusion, the low permeability of compound with SEQ ID NO 9 shows that the transport mechanism takes places through an active mechanism.

### Example 32: Stability of the shuttles in human serum

One of the major advantages of the shuttles of this invention is that unlike the vast majority of peptides composed of L-amino acids (which are rapidly metabolized by a series of enzymes present in the serum of the blood, thus limiting their therapeutic effects), these particular sequences and the cyclic nature of the peptides significantly increase their half-life in serum.

Regarding to the stability study in human serum of the BBB-shuttle (examples 1, 2, 3 and 5), the peptides were incubated at a concentration of 400 µM in buffer HBSS at 37 °C in the presence of 90% human serum. At a range of times, 100 µL aliquots were collected to which methanol was added in order to precipitate the serum proteins. Samples were centrifuged, filtered and analyzed by HPLC-UV and MALDI-TOF MS to determine the degree of degradation of the BBB-shuttle. No degradation in 24 h was observed for the derivatives bearing two disulfide bridges (example 1) or an amide bond (example 3). The half life of the derivative bearing a single disulfide bond was 3 h (examples 2), whereas that of the linear version was no more than 8 min.

### Example 33: Lack of acute toxicity.

1) In zebra fish: The assay was performed by Neuron Bio (Granada). The protocol was based on the assessment of the acute toxicity of substances on zebra fish (Danio rerio) using a static method. This short-term toxicity assay method replicated the guidelines of the document OECD Draft Guideline for the study of chemicals, Fish Embryo Toxicity (FET) Test (30 May, 2006). 5 concentrations were evaluated (4.6, 10, 22, 46 and 100 mg/L) in water containing 0.1% DMSO. A control group (vehicle: water and 0.1 % DMSO) and a group with 3,4-dicloroaniline were set up to make sure test conditions were reproducible.
   None of the lethal parameters studied in the embrios treated with the compound with SEQ ID NO:9 was different from those treated with the vehicle. The parameteres of the embrios treated with 3,4-dichloroaniline were in the established range.
2) In mice: The lack of toxicity of compound with SEQ ID NO:9 compared to apamin was determined by the lethality after intracaudal injection in mice. Apamin (SEQ ID NO:16) was dissolved in phosphate buffer saline (vehicle) so that 200 µL would contain 50 nmol of this peptide (corresponding to its LD₅₀). Compound with SEQ ID NO:9 was dissolved in the same buffer so that 200 µL would contain 1200 nmol of this peptide (near the solubility limit). A first group of six mice (male, 20±2 g, Swiss white mice) were injected with 200 µL of apamin. A second group of six mice were injected with 200 µL of compound with SEQ ID NO:9. A third group of mice were injected with 200 µL of vehicle. The animals were observed at 0.5, 1 5, 10 and 24 hours.

All the animals injected with apamin showed convulsions during the first hours and two of them died. Conversely, no animal treated with compound with SEQ ID NO:9 died or showed any envenomation symptoms and they behaved as the control group injected with the vehicle.

In summary, the examples 1, 2, 3 and 4 are peptides lacking the acute toxicity of apamin with a surprisingly high transport across the blood-brain barrier, remarkably higher than the one of apamin, and exceptional stability to proteases, unexpected in all-L-amino-acid peptides, which may be used as BBB-shuttles.

### REFERENCES CITED IN THE APPLICATION

- C. Devaux et al., European Journal of Biochemistry, 1995, vol. 231, pp. 544-550
- J.P. Vincent et al., Biochemistry, 1975, vol. 14, pp.2521-2525
- L.W. Cosand et al., Proc Natl Acad Sci USA, 1977, vol .74, pp.2771-2775
- M. Amblard, et al., Molecular Biotechnology 2006, Vol. 33, p. 239-254
- C. Soto et al., Nature Medicine 1998, vol.4, p.822-826
- J.H. Lee et al. Eur. J. Biochem. 2001. vol. 268. pp. 2004-2012
- E. Kaiser et al., Anal. Biochem. 1970, vol. 34, pp. 595-598
- A. Madder et al., Eur. J. Org. Chem. 1999, pp. 2787-2791
- S.F.M. van Dongen et al., Bioconjugate Chem. 2009, vol. 20, pp. 20-23
- G.L. Verdine, J. Helinski. Methods in Enzymology. 2012. vol. 503. pp. 3-33
- N.W. Whelan et al. Tetrahedron letters. 2004. vol. 20. pp. 9545-9547
- J. Malcor et al. J. Med. Chem. 2006. vol 55. pp. 2227-2241
- R. Cecchelli et al. PloS One. 2017. Vol 9, pp. e99733
- Prades et al. Biomaterials 2012, vol 33, pp. 7194-7205
- B. T: Brazil et al., in Journal of Biological Chemistry, 1997, vol. 72, pp.5105-5111

## Claims

1. A peptidic compound of formula
R₁-(V)ₖ-P-(W)ₛ-Y (I)
wherein:
R₁ is the group attached to the N-terminal of the first amino acid of the sequence P, via the biradical V, and is selected from the group consisting of hydrogen, CH₃C(=O)-, and maleimide;
V is a biradical selected from the group consisting of -NH-(CH₂)ᵣ-C(=O)-, -NH-CH-((CH₂)ᵣNH₂)-C(=O)-, -C(=O)-(CH₂)ᵣ-C(=O)-, -S-(CH₂)ᵣ-, -S-(CH₂)r-C(=O)-, -O-(CH₂)ᵣ-, -S-CH₂-CH(NH₂)-C(=O)-, -O-(CH₂)ᵣ-C(=O)-, -(CH₂)ᵣC(=O)-, -NH-O-CH₂-C(=O)-NH-(CH₂), -CH(NH₂)-C(=O)-, and -C≡C-(CH₂)ᵣC(=O)-;
the biradical V being attached to R₁ and to the N of the sequence P as follows: R₁-NH-(CH₂)ᵣ-C(=O)-N(H)ₘ-, R₁-NH-CH-((CH₂)ᵣNH₂)-C(=O)-N(H)ₘ, R₁-C(=O)-(CH₂)ᵣC(=O)-N(H)ₘ-, R₁-S-(CH₂)ᵣN(H)ₘ-, R₁-S-(CH₂)ᵣ-C(=O)-N(H)ₘ-, R₁-O-(CH₂)ᵣ-N(H)ₘ-, R₁-S-CH₂-CH(NH₂)-C(=O)-N(H)ₘ, R₁-O-(CH₂)ᵣ-C(=O)-N(H)ₘ-, R₁-(CH₂)ᵣ-C(=O)-N(H)ₘ, R₁-NH-O-CH₂-C(=O)-NH-(CH₂)ᵣ-CH(NH₂)-C(=O)-N(H)ₘ; and R₁ -C≡C-(CH₂)ᵣC(=O)-N(H)ₘ;
P is a biradical of:
(a) a peptide having 9-20 amino acids residues in length having at least an intrapeptide bond which is a disulfide bond, and comprises an amino acid sequence which is:
CKAPETALCAAA (SEQ ID NO:7);
having at least an intrapeptide disulfide bond between cysteines 1 and 9,
or a peptide having 9-11 amino acids residues in length having at least an intrapeptide bond which is a disulfide bond and consists of an amino acid sequence selected from the group consisting of
CKAPETALC (SEQ ID NO:4);
CKAPETALCA (SEQ ID NO:5); and
CKAPETALCAA (SEQ ID NO:6); having at least an intrapeptide disulfide bond between cysteines 1 and 9; or alternatively,
(b) P has 16 amino acid residues and comprises the amino acid sequence
CNCKAPETALCAAACH (SEQ ID NO:9)
with an intrapeptide disulfide bond between the first and third cysteine which are cysteines 1 and 11, and between the second and the fourth cysteine which are cysteine 3 and 15, or alternatively;
(c) P comprises the amino acid sequence
DapKAPETALD (SEQ ID NO:12)
with an intrapeptide bond between the Dap and D which is an amide bond;
W is a biradical selected from the group consisting of -NH-(CH₂)ᵣ-C(=O)-, and -NH-CH((CH₂)ᵣNH₂)-C(=O)-;
the biradical W being attached to the C=O of the sequence P and to Y as follows: -C(=O)-NH-(CH₂)ᵣ-C(=O)-Y, or -C(=O)-NH-CH((CH₂)ᵣNH₂)-C(=O)-Y;
Y is the group attached to the C-terminal of the last amino acid of the sequence P, and is selected from the group consisting of -NH₂, -OH, -OR₂, and -NHR₂;
R₂ is a radical selected from the group consisting of (C₁-C₆)-alkyl and (CH₂)₂-NH-C(=O)-CH₂-O-NH₂;
r is an integer from 1 to 5;
k is an integer from 0 to 1;
m is an integer from 0 to 1;
s is an integer from 0 to 1;
with the proviso that:
when the biradical V is -C(=O)(CH₂)ᵣC(=O)-, then R₁ is hydrogen;
when the N of the amino acid of the sequence P to which is attached the biradical V is a biradical -NH-, then m is 1, and when the N of the amino acid of the sequence P to which is attached the biradical -V- is a biradical -N-, then m is 0; and
when R₁ is maleimide then the biradical V is -(CH₂)ᵣ-C(=O)-.

2. The peptidic compound according to claim 1, wherein P comprises the amino acid sequence CKAPETALCAAA (SEQ ID NO:7)

3. The peptidic compound according to any of the claims 1-2, wherein either k is 0 and R₁ is hydrogen, or alternatively, s is 0 and Y is NH₂.

4. A construct of formula (II) or a pharmaceutically acceptable salt thereof,
(R₁)ₑ-(Z)_{q1}-(V)ₖ-P-(W)ₛ-(Z)_{q2}-(Y)_{f}, (II)
wherein:
P is a biradical of the peptide P as defined in any of the claims 1-3, or alternatively, the amino acid sequence KAPETAL(SEQ ID NO:10); R₁, W, Y, k, and s are as defined in any of the claims 1-3;
V is as defined in any of the claims 1-3, or alternatively is selected from the group consisting of V₁, V₂, and V₃,
V₁ being 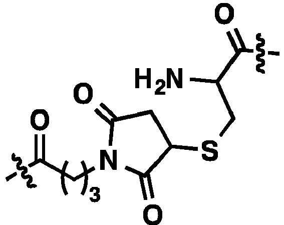
V₂ being 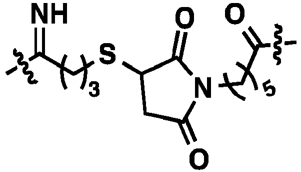
and V₃ being 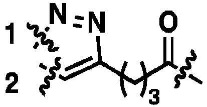 wherein bonds 1 and 2 are both connected to the side-chain of a lysine from the protein or antibody cargo;
Z is a radical independently selected of a biologically active substance which is an active pharmaceutical ingredient capable of forming an amide bond, an ester bond, a disulfide bond, a thioether bond, an oxime bond, an amine bond, a 1,4-disubstituted 1,2,3-triazole bond (triazole bond), or an hydrazone bond with V or an amide bond or an ester bond with W, and is selected from the group consisting of antiretroviral agents, anticancer agents, antipsychotic agents, antineurodegenerative agents, a therapeutic protein for protein replacement therapy, and antiepileptic agents; or a substance for use in a diagnostic method selected from the group consisting of 5(6)-carboxyfluorescein, biotin, sulforhodamine B, Quantum dots, and superparamagnetic iron oxide nanoparticles (Spion);
said substance being substantially unable to cross the BBB by itself;
q1 and q2 are integers from 0 to 2, being q1 and/or q2 different than 0 and the sum of q1 and q2 being 1 or 2;
e and f are integers from 0 to 1;
with the proviso that:
when q1 is 2 and the biradical V has a non-terminal nitrogen, then one Z is attached to the N-terminal of the first amino acid of the sequence P via the biradical V, and the other Z is attached to the non-terminal nitrogen of the biradical V, and k is 1;
when q2 is 2 and the biradical W has a non-terminal nitrogen, then one Z is attached to the C=O terminal of the last amino acid of the sequence P, via the biradical W, and the other Z is attached to the non-terminal nitrogen of the biradicals W and s is 1; and
when q1 is 2 then e is 0, when q2 is 2 then f is 0,
when q1 is 1 then Z is attached to the N-terminal of the first amino acid of the sequence P, optionally via the biradical V, and e is 0; and
when q2 is 1 then Z is attached to the C=O terminal of the last amino acid of the sequence P, optionally via the biradical W, and f is 0.

5. The construct according to claim 4, wherein V is as defined in any of the claims 1-3.

6. The construct according to any of the claims 4-5, wherein the biradical of the peptide P is
CKAPETALCAAA (SEQ ID NO:7)

7. The construct according to any of the claims 4-6, wherein Z is a radical of an active pharmaceutical ingredient capable of forming an amide bond, an ester bond, a disulfide bond, a thioether bond, an oxime bond, an amine bond, a 1,4-disubstituted 1,2,3-triazole bond (triazole bond), or an hydrazone bond with V or an amide bond or an ester bond with W, and is selected from the group consisting of antiretroviral agents, anticancer agents, antipsychotic agents, antineurodegenerative agents, a therapeutic protein for protein replacement therapy, and antiepileptic agents.

8. The construct according to claim 7, wherein the pharmaceutical active agent is selected from the group consisting of an antibody, L-dopamine and LPFFD (SEQ ID NO:11).

9. The construct according to any of the claims 4-6, wherein Z is a radical of a compound selected from the group consisting of 5(6)-carboxyfluorescein, biotin, sulforhodamine B, Quantum dots, and superparamagnetic iron oxide nanoparticles (Spion).

10. The construct according to any of the claims 4-6, wherein the construct is selected from the group consisting of:
L-Dopa-(Dap)KAPETALD-NH₂;
H-(Dap)KAPETALDKQIEIKKFK-NH₂;
Cetuximab-V₃-CNCKAPETALCAAACH-NH₂;
Cetuximab-V₂-DapKAPETALD-NH₂;
Quantum dot-PEG-NH-V₁-DapKAPETALD-NH₂; and
Gold nanoparticle-CDapKAPETALD-NH₂,
V₁ being 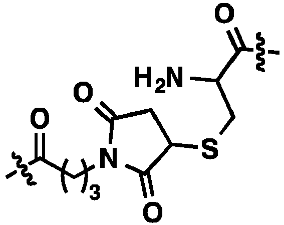
V2 being 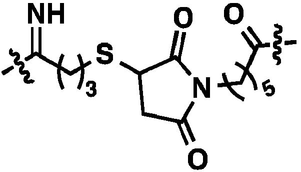
and V₃ being 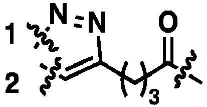 wherein bonds 1 and 2 are both connected to the side-chain of a lysine from the protein or antibody cargo.

11. A pharmaceutical composition or a composition for diagnostic purposes comprising a therapeutically or diagnostic effective amount of the construct as defined in any of the claims 4-10, together with appropriate amounts of pharmaceutically acceptable carriers or excipients and/or acceptable for diagnosis.

12. A construct of formula (II) as defined in any of the claims 4-6, wherein Z is a radical of a biologically active substance, for use as a medicament wherein the biologically active substance is an active pharmaceutical ingredient capable of forming an amide bond, an ester bond, a disulfide bond, a thioether bond, an oxime bond, an amine bond, a 1,4-disubstituted 1,2,3-triazole bond (triazole bond), or an hydrazone bond with V or an amide bond or an ester bond with W, and is selected from the group consisting of antiretroviral agents, anticancer agents, antipsychotic agents, antineurodegenerative agents, a therapeutic protein for protein replacement therapy, and antiepileptic agents;.

13. A construct of formula (II) as defined in any of the claims 4-6, wherein Z is a superparamagnetic iron oxide nanoparticle or a gadolinium complex, for use as a contrast agent for a magnetic resonance imaging diagnostic method

14. A construct of formula (II) as defined in any of the claims 4-6, wherein, Z is selected from the group consisting of 5(6)-carboxyfluorescein, Texas red, rhodamin, sulforhodamine, and quantum dots, for use as a fluorescent probe for an image diagnostic method.

## Patentansprüche

1. Eine peptidische Verbindung der Formel
R₁-(V)ₖ-P-(W)ₛ-Y (I)
wobei:
R₁ die über das Biradikal V an den N-Terminus der ersten Aminosäure der Sequenz P gebundene Gruppe ist und ausgewählt ist aus der Gruppe bestehend aus Wasserstoff, CH₃C(=O)- und Maleimid;
V ein Biradikal ist, das ausgewählt ist aus der Gruppe bestehend aus -NH-(CH₂)ᵣ-C(=O)-, -NH-CH-((CH₂)ᵣNH₂)-C(=O)-, -C(=O)-(CH₂)ᵣ-C(=O)-, -S-(CH₂)ᵣ-, -S-(CH₂)ᵣ-C(=O)-, -O-(CH₂)ᵣ-, -S-CH₂-CH(NH₂)-C(=O)-, -O-(CH₂)ᵣ-C(=O)-, -(CH₂)ᵣ-C(=O)-, -NH-O-CH₂-C(=O)-NH-(CH₂)ᵣ-CH(NH₂)-C(=O)-, und -C≡C-(CH₂)ᵣC(=O)-
das Biradikal V an R₁ und an den N der Sequenz P wie folgt gebunden ist: R₁-NH-(CH₂)ᵣ-C(=O)-N(H)ₘ-, R₁-NH-CH-((CH₂)ᵣNH₂)-C(=O)-N(H)ₘ, R₁-C(=O)-(CH₂)ᵣ-C(=O)-N(H)ₘ-, R₁-S-(CH₂)ᵣ-N(H)ₘ-, R₁-S-(CH₂)ᵣ-C(=O)-N(H)ₘ-, R₁-O-(CH₂)ᵣ-N(H)ₘ-, R₁-S-CH₂-CH(NH₂)-C(=O)-N(H)ₘ, R₁-O-(CH₂)ᵣ-C(=O)-N(H)ₘ-, R₁-(CH₂)ᵣ-C(=O)-N(H)ₘ, R₁-NH-O-CH₂-C(=O)-NH-(CH₂)ᵣ-CH(NH₂)-C(=O)-N(H)ₘ; und R₁-C≡C-(CH₂)ᵣC(=O)-N(H)ₘ;
P ein Biradikal ist von:
(a) einem Peptid mit einer Länge von 9-20 Aminosäureresten mit mindestens einer Intrapeptidbindung, welche eine Disulfidbindung ist und eine Aminosäuresequenz umfasst, die wie folgt ist:
CKAPETALCAAA (SEQ ID NO:7);
das mindestens eine Intrapeptiddisfulfidbindung zwischen den Cysteinen 1 und 9 hat,
oder einem Peptid mit einer Länge von 9-11 Aminosäureresten mit mindestens einer Intrapeptidbindung, welche eine Disulfidbindung ist und aus einer Aminosäuresequenz besteht, die ausgewählt ist aus der Gruppe bestehend aus
CKAPETALC (SEQ ID NO:4);
CKAPETALCA (SEQ ID NO:5); und
CKAPETALCAA (SEQ ID NO:6); das mindestens eine Intrapeptiddisfulfidbindung zwischen den Cysteinen 1 und 9 hat; oder, ersatzweise,
(b) P 16 Aminosäurereste hat und folgende Aminosäuresequenz umfasst
CNCKAPETALCAAACH (SEQ ID NO:9)
mit einer Intrapeptiddisulfidbindung zwischen dem ersten und dem dritten Cystein, welche die Cysteine 1 und 11 sind, und zwischen dem zweiten und dem vierten Cystein, welche die Cysteine 3 und 15 sind, oder, ersatzweise;
(c) P folgende Aminosäuresequenz umfasst
DapKAPETALD (SEQ ID NO:12)
mit einer Intrapeptidbindung zwischen der Dap und D, welche eine Amidbindung ist;
W ein Biradikal ist, das ausgewählt aus der Gruppe bestehend aus -NH-(CH₂)ᵣ-C(=O)-, und -NH-CH((CH₂)ᵣNH₂)-C(=O)- ist;
wobei das Biradikal W an das C=O der Sequenz P und an Y wie folgt gebunden ist: -C(=O)-NH-(CH₂)ᵣ-C(=O)-Y, oder -C(=O)-NH-CH((CH₂)ᵣNH₂)-C(=O)-Y;
Y die Gruppe ist, die an den C-Terminus der letzten Aminosäure der Sequenz P gebunden ist, und ausgewählt ist aus der Gruppe bestehend aus -NH₂, -OH, -OR₂, und -NHR₂;
R₂ ein Radikal ist, das ausgewählt aus der Gruppe bestehend aus (C₁-C₆)-Alkyl und (CH₂)₂-NH-C(=O)-CH₂-O-NH₂ ist;
r eine Ganzzahl von 1 bis 5 ist;
k eine Ganzzahl von 0 bis 1 ist;
m eine Ganzzahl von 0 bis 1 ist;
s eine Ganzzahl von 0 bis 1 ist;
mit der Maßgabe, dass
wenn das Biradikal V-C(=O)(CH₂)ᵣC(=O)- ist, dann ist R₁ Wasserstoff;
wenn der N der Aminosäure der Sequenz P, an den das Biradikal V gebunden ist, ein Biradikal -NH- ist, dann ist m = 1 und wenn der N der Aminosäure der Sequenz P, an den das Biradikal -V- gebunden ist, ein Biradikal -N- ist, dann ist m = 0; und
wenn R₁ Maleimid ist, dann ist das Biradikal V-(CH₂)ᵣ-C(=O)-.

2. Die peptidische Verbindung nach Anspruch 1, wobei P folgende Aminosäuresequenz umfasst: CKAPETALCAAA (SEQ ID NO:7).

3. Die peptidische Verbindung nach einem der Ansprüche 1 bis 2, wobei k = 0 und R₁ Wasserstoff ist oder, ersatzweise, s = 0 und Y = NH₂ ist.

4. Ein Konstrukt der Formel (II) oder ein pharmazeutisch akzeptables Salz davon,
(R₁)ₑ-(Z)_{q1}-(V)ₖ-P-(W)ₛ-(Z)_{q2}-(Y)_{f}, (II)
wobei:
P ein Biradikal des Peptids wie in einem der Ansprüche 1 bis 3 definiert ist oder, ersatzweise, die Aminosäuresequenz KAPETAL (SEQ ID NO: 10); R₁, W, Y, k und s wie in einem der Ansprüche 1 bis 3 definiert sind;
V wie in einem der Ansprüche 1 bis 3 definiert ist oder, ersatzweise, ausgewählt ist aus der Gruppe bestehend aus V₁, V₂, und V₃, wobei diese wie folgt sind:
V₁ 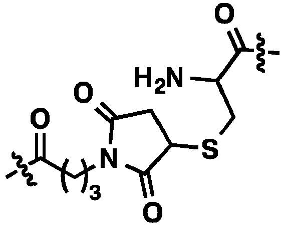
V₂ 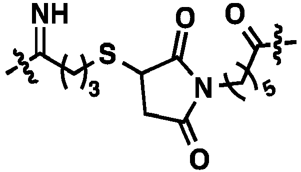
und V₃ 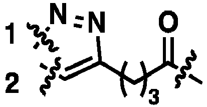 wobei die Bindungen 1 und 2 jeweils an die Seitenkette von einem Lysin von dem Protein oder Antikörperladung gebunden sind;
Z ein Radikal ist, das unabhängig ausgewählt ist aus einer biologisch aktiven Substanz, welche ein aktiver pharmazeutischer Wirkstoff ist, der eine Amidbindung, eine Esterbindung, eine Disulfidbindung, eine Thioetherbindung, eine Oximbindung, eine Aminbindung, eine 1,4-disubstituierte 1,2,3-Triazolbindung (Triazolbindung) oder eine Hydrazonbindung mit V oder eine Amidbindung oder eine Esterbindung mit W bilden kann, und ausgewählt ist aus der Gruppe bestehend aus antiretroviralen Mitteln, Antikrebsmitteln, Antipsychotika, antineurodegenerativen Mitteln, einem therapeutischen Protein für Protein-Ersatztherapie, und Antiepileptika; oder einer Substanz zur Verwendung in einem diagnostischen Verfahren ausgewählt aus der Gruppe bestehend aus 5(6)-Carboxyfluorescein, Biotin, Sulforhodamin B, Quantenpunkten, und superparamagnetischen Eisenoxidnanopartikeln (Spion); wobei die Substanz selbst im Wesentlichen die Blut-Hirn-Schranke nicht durchqueren kann;
q1 und q2 Ganzzahle von 0 bis 2 sind, wobei q1 und/oder q2 nicht 0 sind und die Summe von q1 und q2 = 1 oder 2 ist;
e und f Ganzzahle von 0 bis 1 sind;
mit der Maßgabe, dass
wenn q1 = 2 ist und das Biradikal V einen nicht terminalen Stickstoff hat, dann ist ein Z an den N-Terminus der ersten Aminosäure der Sequenz P über das Biradikal V gebunden und ist das andere Z an den nicht terminalen Stickstoff des Biradikals V gebunden und k = 1;
wenn q2 = 2 ist und das Biradikal W einen nicht terminalen Stickstoff hat, dann ist ein Z an den C=O-Terminus der letzten Aminosäure der Sequenz P über das Biradikal W gebunden und ist das andere Z an den nicht terminalen Stickstoff der Biradikale W gebunden und s = 1; und
wenn q1 = 2, dann ist e = 0, wenn q2 = 2, dann ist f = 0,
wenn q1 = 1, dann ist Z an den N-Terminus der ersten Aminosäure der Sequenz P, wahlweise über das Biradikal V, gebunden und e = 0; und
wenn q2 = 1, dann ist Z an den C=O-Terminus der letzten Aminosäure der Sequenz P, wahlweise über das Biradikal W, gebunden und f = 0.

5. Das Konstrukt nach Anspruch 4, wobei V wie in einem der Ansprüche 1 bis 3 definiert ist.

6. Das Konstrukt nach einem der Ansprüche 4 bis 5, wobei das Biradikal des Peptids P wie folgt ist
CKAPETALCAAA (SEQ ID NO:7).

7. Das Konstrukt nach einem der Ansprüche 4 bis 6, wobei Z ein Radikal von einem aktiven pharmazeutischen Wirkstoff ist, der eine Amidbindung, eine Esterbindung, eine Disulfidbindung, eine Thioetherbindung, eine Oximbindung, eine Aminbindung, eine 1,4-disubstituierte 1,2,3-Triazolbindung (Triazolbindung), oder eine Hydrazonbindung mit V oder eine Amidbindung oder eine Esterbindung mit W bilden kann, und aus der Gruppe bestehend aus Antiretroviralmitteln, Antikrebsmitteln, Antipsychotika, antineurodegenerativen Mitteln, einem therapeutischen Protein zur Protein-Ersatztherapie, und Antiepileptika ausgewählt ist.

8. Das Konstrukt nach Anspruch 7, wobei der pharmazeutische Wirkstoff ausgewählt aus der Gruppe bestehend aus einem Antikörper, L-Dopamin und LPFFD (SEQ ID NO:11) ist.

9. Das Konstrukt nach einem der Ansprüche 4 bis 6, wobei Z ein Radikal von einer Verbindung ist, die ausgewählt ist aus der Gruppe bestehend aus 5(6)-Carboxyfluorescein, Biotin, Sulforhodamin B, Quantenpunkten, und superparamagnetischen Eisenoxidnanopartikeln (Spion).

10. Das Konstrukt nach einem der Ansprüche 4 bis 6, wobei das Konstrukt ausgewählt ist aus der Gruppe bestehend aus:
L-Dopa-(Dap)KAPETALD-NH₂;
H-(Dap)KAPETALDKQIEIKKFK-NH₂;
Cetuximab-V₃-CNCKAPETALCAAACH-NH₂;
Cetuximab-V₂-DapKAPETALD-NH₂;
Quantenpunkt-PEG-NH-V₁-DapKAPETALD-NH₂; und
Goldnanopartikel-CDapKAPETALD-NH₂,
V₁ 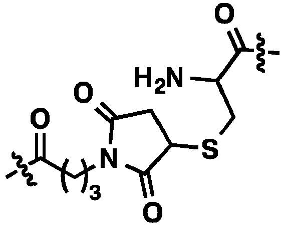
V₂ 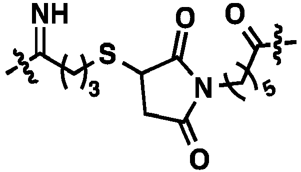
und V₃ 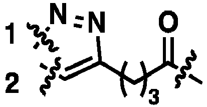 wobei die Bindungen 1 und 2 jeweils an die Seitenkette von einem Lysin von dem Protein oder Antikörperladung gebunden sind.

11. Eine pharmazeutische Zusammensetzung oder eine Zusammensetzung für diagnostische Zwecke umfassend eine therapeutisch oder diagnostisch wirksame Menge des Konstrukts wie in einem der Ansprüche 4 bis 10 definiert, zusammen mit geeigneten Mengen an pharmazeutisch akzeptablen Trägern oder Hilfsstoffen und/oder die akzeptabel für Diagnose sind.

12. Ein Konstrukt der Formel (II) wie in einem der Ansprüche 4 bis 6 definiert, wobei Z ein Radikal von einer biologisch aktiven Substanz ist, zur Verwendung als Arzneimittel, wobei die biologisch aktive Substanz ein aktiver pharmazeutischer Wirkstoff ist, der eine Amidbindung, eine Esterbindung, eine Disulfidbindung, eine Thioetherbindung, eine Oximbindung, eine Aminbindung, eine 1,4-disubstituierte 1,2,3-Triazolbindung (Triazolbindung), oder eine Hydrazonbindung mit V oder eine Amidbindung oder eine Esterbindung mit W bilden kann, und aus der Gruppe bestehend aus Antiretroviralmitteln, Antikrebsmitteln, Antipsychotika, antineurodegenerativen Mitteln, einem therapeutischen Protein zur Protein-Ersatztherapie, und Antiepileptika ausgewählt ist.

13. Ein Konstrukt der Formel (II) wie in einem der Ansprüche 4 bis 6 definiert, wobei Z eine superparamagentische Eisenoxidnanopartikel oder ein Gadoliniumkomplex ist, zur Verwendung als Kontrastmittel für ein diagnostisches Verfahren der Magnetresonanztomographie.

14. Ein Konstrukt der Formel (II) wie in einem der Ansprüche 4 bis 6 definiert, wobei Z ausgewählt ist aus der Gruppe bestehend aus 5(6)-Carboxyfluorescein, Texasrot, Rhodamin, Sulforhodamin, und Quantenpunkten, zur Verwendung als fluoreszierender Marker für ein bilddiagnostisches Verfahren.

## Revendications

1. Un composé peptidique de formule
R₁-(V)ₖ-P-(W)ₛ-Y (I)
dans laquelle :
R₁ est le groupe lié à l'extrémité N-terminale du premier acide aminé de la séquence P, via le biradical V, et est choisi dans le groupe constitué d'hydrogène, CH₃C(=O)-, et maléimide ;
V est un biradical choisi dans le groupe constitué de -NH-(CH₂)ᵣ-C(=O)-, -NH-CH-((CH₂)ᵣNH₂)-C(=O)-, -C(=O)-(CH₂)ᵣ-C(=O)-, -S-(CH₂)ᵣ-, -S-(CH₂)ᵣ-C(=O)-, -O-(CH₂)ᵣ-, - S-CH₂-CH(NH₂)-C(=O)-, -O-(CH₂)ᵣ-C(=O)-, -(CH₂)ᵣ-C(=O)-, -NH-O-CH₂-C(=O)-NH-(CH₂)ᵣ-CH(NH₂)-C(=O)-, et
-C≡C-(CH₂)ᵣC(=O)-
le biradical V étant lié à R₁ et au N de la séquence P comme suit : R₁-NH-(CH₂)ᵣ-C(=O)-N(H)ₘ-, R₁-NH-CH-((CH₂)ᵣNH₂)-C(=O)-N(H)ₘ, R₁-C(=O)-(CH₂)ᵣ-C(=O)-N(H)ₘ-, R₁-S-(CH₂)ᵣ-N(H)ₘ-, R₁-S-(CH₂)ᵣ-C(=O)-N(H)ₘ-, R₁-O-(CH₂)ᵣ-N(H)ₘ-, R₁-S-CH₂-CH(NH₂)-C(=O)-N(H)ₘ, R₁-O-(CH₂)ᵣ-C(=O)-N(H)ₘ-, R_{d}(CH₂)ᵣ-C(=O)-N(H)ₘ, R₁-NH-O-CH₂-C(=O)-NH-(CH₂)ᵣ-CH(NH₂)-C(=O)-N(H)ₘ ; et R₁-C≡C-(CH₂)ᵣC(=O)-N(H)ₘ;
P est un biradical de :
(a) un peptide ayant une longueur de 9 à 20 résidus d'acides aminés ayant au moins une liaison intrapeptidique qui est une liaison disulfure, et comprend une séquence d'acides aminés qui est :
CKAPETALCAAA (SEQ ID NO:7) ;
ayant au moins une liaison disulfure intrapeptidique entre les cystéines 1 et 9,
ou un peptide ayant une longueur de 9 à 11 résidus d'acides aminés ayant au moins une liaison intrapeptidique qui est une liaison disulfure, et constitué d'une séquence d'acides aminés qui est choisie dans le groupe constitué de :
CKAPETALC (SEQ ID NO:4) ;
CKAPETALCA (SEQ ID NO:5) ; et
CKAPETALCAA (SEQ ID NO:6) ; ayant au moins une liaison disulfure intrapeptidique entre les cystéines 1 et 9 ; ou, alternativement,
(b) P a 16 résidus acides aminés et comprend la séquence d'acides aminés
CNCKAPETALCAAACH (SEQ ID NO:9)
avec une liaison disulfure intrapeptidique entre la première et la troisième cystéine qui sont les cystéines 1 et 11, et entre la deuxième et la quatrième cystéine, qui sont les cystéines 3 et 15, ou, alternativement ;
(C) P comprend la séquence d'acides aminés
DapKAPETALD (SEQ ID NO:12)
avec une liaison intrapeptidique entre le Dap et le D qui est une liaison amide ;
W est un biradical choisi dans le groupe constitué de -NH-(CH₂)ᵣ-C(=O)-, et-NH-CH((CH₂)ᵣNH₂)-C(=O)- ;
le biradical W étant lié au C=O de la séquence P et à Y comme suit : -C(=O)-NH-(CH₂)ᵣ-C(=O)-Y, ou -C(=O)-NH-CH((CH₂)ᵣNH₂)-C(=O)-Y ;
Y est le groupe lié à l'extrémité C-terminale du dernier acide aminé de la séquence P, et est choisi dans le groupe constitué de -NH₂, -OH, -OR₂, et -NHR₂ ;
R₂ est un radical choisi dans le groupe constitué d'alkyle en (C₁-C₆) et (CH₂)₂-NH-C(=O)-CH₂-O-NH₂ ;
r est un nombre entier allant de 1 à 5 ;
k est un nombre entier allant de 0 à 1 ;
m est un nombre entier allant de 0 à 1 ;
s est un nombre entier allant de 0 à 1 ;
à condition que :
lorsque le biradical V est -C(=O)(CH₂)ᵣC(=O)-, alors R₁ est hydrogène ;
lorsque le N de l'acide aminé de la séquence P auquel est lié le biradical V est un biradical -NH-, alors m vaut 1, et quand le N de l'acide aminé de la séquence P auquel est lié le biradical -V- est un biradical -N-, alors m vaut 0 ; et
lorsque R₁ est un maléimide, alors le biradical V est -(CH₂)ᵣ-C(=O)-.

2. Le composé peptidique selon la revendication 1, dans lequel P comprend la séquence d'acides aminés CKAPETALCAAA (SEQ ID NO:7).

3. Le composé peptidique selon l'une quelconque des revendications 1 à 2, dans lequel ou bien k est 0 et R₁ est hydrogène ou, alternativement, s est 0 et Y est NH₂.

4. Une construction de formule (II) ou un sel pharmaceutiquement acceptable de celle-ci,
(R₁)ₑ-(Z)_{q1}-(V)ₖ-P-(W)ₛ-(Z)_{q2}-(Y)_{f}, (II)
dans laquelle :
P est un biradical du peptide P tel que défini dans l'une quelconque des revendications 1 à 3 ou, alternativement, la séquence d'acides aminés KAPETAL (SEQ ID NO:10) ;
R₁, W, Y, k, et s sont tel que définis dans l'une quelconque des revendications 1 à 3 ;
V est tel que défini dans l'une quelconque des revendications 1 à 3 ou, alternativement, est choisi dans le groupe constitué de V₁, V₂, et V₃,
V₁ étant 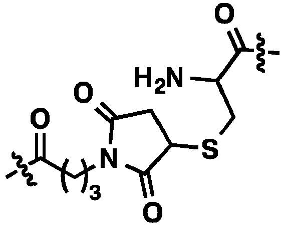
V₂ étant 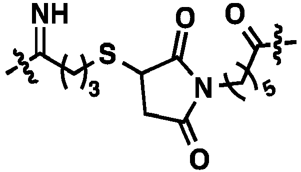
et V₃ étant 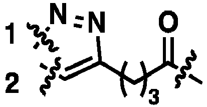 où les liaisons 1 et 2 sont toutes deux liées à la chaîne latérale d'une lysine de la protéine ou une cargaison d'anticorps ;
Z est un radical indépendamment choisi d'une substance biologiquement active qui est un ingrédient pharmaceutique actif capable de former une liaison amide, une liaison ester, une liaison disulfure, une liaison thioéther, une liaison oxime, une liaison amine, une liaison 1,2,3-triazole 1,4-disubstituée (liaison triazole), ou une liaison hydrazone avec V ou une liaison amide ou une liaison ester avec W, et est choisi dans le groupe constitué par les agents antirétroviraux, les agents anticancéreux, les agents antipsychotiques, les agents antineurodégénératifs, une protéine thérapeutique pour thérapie de remplacement des protéines, et des agents antiépileptiques ; ou une substance pour l'utilisation dans un procédé diagnostic choisi dans le groupe constitué de la 5(6)-carboxyfluorescéine, la biotine, la sulforhodamine B, des points quantiques, et des nanoparticules d'oxyde de fer superparamagnétiques (Spion) ;
ladite substance étant essentiellement incapable de traverser la barrière hémato-encéphalique par elle même ;
q1 et q2 sont des nombres entiers allant de 0 à 2, q1 et/ou q2 étant différents de 0 et la somme de q1 et q2 étant 1 ou 2 ;
e et f sont des nombres entiers allant de 0 à 1 ;
à condition que :
lorsque q1 est 2 et le biradical V a un azote non terminal, alors un Z est lié à l'extrémité N-terminale du premier acide aminé de la séquence P via le biradical V, et l'autre Z est lié à l'azote non terminal du biradical V, et k est 1 ;
lorsque q2 est 2 et le biradical W a un azote non terminal, alors un Z est lié à l'extrémité C=O-terminale du dernier acide aminé de la séquence P via le biradical W, et l'autre Z est lié à l'azote non terminal des biradicaux W, et s est 1 ; et
lorsque q1 est 2, alors e est 0, lorsque q2 est 2, alors f est 0,
lorsque q1 est 1, alors Z est lié à l'extrémité N-terminale du premier acide aminé de la séquence P, facultativement via le biradical V, et e est 0 ; et
lorsque q2 est 1, alors Z est lié à l'extrémité C=O-terminale du dernier acide aminé de la séquence P, facultativement via le biradical W, et f est 0.

5. La construction selon la revendication 4, dans laquelle V est tel que défini dans l'une quelconque des revendications 1 à 3.

6. La construction selon l'une quelconque des revendications 4 à 5, dans laquelle le biradical du peptide P est
CKAPETALCAAA (SEQ ID NO:7).

7. La construction selon l'une quelconque des revendications 4 à 6, dans laquelle Z est un radical d'un ingrédient pharmaceutique actif capable de former une liaison amide, une liaison ester, une liaison disulfure, une liaison thioéther, une liaison oxime, une liaison amine, une liaison 1,2,3-triazole 1,4-disubstituée (liaison triazole), ou une liaison hydrazone avec V ou une liaison amide ou une liaison ester avec W, et est choisi dans le groupe constitué par les agents antirétroviraux, les agents anticancéreux, les agents antipsychotiques, les agents antineurodégénératifs, une protéine thérapeutique pour thérapie de remplacement des protéines, et des agents antiépileptiques.

8. La construction selon la revendication 7, dans laquelle l'agent actif pharmaceutique est choisi dans le groupe constitué d'un anticorps, L-dopamine et LPFFD (SEQ ID NO:11).

9. La construction selon l'une quelconque des revendications 4 à 6, dans laquelle Z est un radical d'un composé choisi dans le groupe constitué de la 5(6)-carboxyfluorescéine, la biotine, la sulforhodamine B, des points quantiques, et des nanoparticules d'oxyde de fer superparamagnétiques (Spion).

10. La construction selon l'une quelconque des revendications 4 à 6, dans laquelle la construction est choisie dans le groupe constitué de :
L-Dopa-(Dap)KAPETALD-NH₂ ;
H-(Dap)KAPETALDKQIEIKKFK-NH₂ ;
Cetuximab-V₃-CNCKAPETALCAAACH-NH₂ ;
Cetuximab-V₂-DapKAPETALD-NH₂ ;
Point quantique-PEG-NH-V₁-DapKAPETALD-NH₂ ; et
Nanoparticule d'or-CDapKAPETALD-NH₂,
V₁ étant 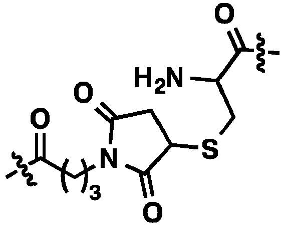
V₂ étant 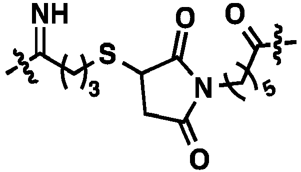
et V₃ étant 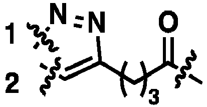 où les liaisons 1 et 2 sont toutes deux liées à la chaîne latérale d'une lysine de la protéine ou une cargaison d'anticorps.

11. Une composition pharmaceutique ou une composition à des fins de diagnostic comprenant une quantité thérapeutiquement ou diagnostique efficace de la construction telle que définie dans l'une quelconque des revendications 4 à 10, conjointement avec des quantités appropriées de supports ou excipients pharmaceutiquement acceptables et/ou acceptables pour le diagnostic.

12. Une construction de formule (II) telle que définie dans l'une quelconque des revendications 4 à 6, dans laquelle Z est un radical d'une substance biologiquement active, pour l'utilisation comme un médicament dans laquelle la substance biologiquement active est un ingrédient pharmaceutique actif capable de former une liaison amide, une liaison ester, une liaison disulfure, une liaison thioéther, une liaison oxime, une liaison amine, une liaison 1,2,3-triazole 1,4-disubstituée (liaison triazole), ou une liaison hydrazone avec V ou une liaison amide ou une liaison ester avec W, et est choisie dans le groupe constitué par les agents antirétroviraux, les agents anticancéreux, les agents antipsychotiques, les agents antineurodégénératifs, une protéine thérapeutique pour thérapie de remplacement des protéines, et des agents antiépileptiques.

13. Une construction de formule (II) telle que définie dans l'une quelconque des revendications 4 à 6, dans laquelle Z est une nanoparticule d'oxyde de fer superparamagnétique ou un complexe de gadolinium, pour l'utilisation comme agent de contraste pour une méthode de diagnostic par imagerie par résonance magnétique.

14. Une construction de formule (II) telle que définie dans l'une quelconque des revendications 4 à 6, dans laquelle Z est choisi dans le groupe constitué par la 5(6)-carboxyfluorescéine, le rouge Texas, la rhodamine, la sulforhodamine, et des points quantiques, pour l'utilisation comme sonde fluorescente pour une méthode de diagnostic par image.
